# EUROPEAN PATENT APPLICATION

(11) **EP 2 151 504 A1**
(43) Date of publication of application: **10.02.2010**
(21) Application number: 08161841.5
(22) Date of filing: 05.08.2008
(51) Int. Cl.: C12Q 1/68

(54) **Interferon**

(71) Applicant: Universitätsklinikum Hamburg-Eppendorf, 20246 Hamburg (DE); Institut de Recerca Hospital Universitari Vall d'Hebron, 08035 Barcelona (ES)
(72) Inventor: Martin, Roland, 20251 Hamburg (DE); Comabella Lopez, Manuel, 08800 Barcelona (ES); Montalban Gairin, Xavier, 08034 Barcelona (ES)
(74) Representative: Steglich, Gregor

(57) **Abstract**

Subject of the invention are methods for predicting if a treatment of multiple sklerosis with type I interferon is successful, methods of treatment, microarrays and kits for performing the methods.

## Description

Subject of the invention are methods for predicting if a treatment of multiple sclerosis with type I interferon is successful, methods of treatment, microarrays and kits for performing the methods.

Multiple sclerosis (MS) is an autoimmune condition in which the immune system attacks the central nervous system (CNS), leading to demyelination. It may cause numerous physical and mental symptoms, and often progresses to physical and cognitive disability. MS takes several forms, with new symptoms occurring either in discrete attacks (relapsing forms) or slowly accumulating over time (progressive forms). Most people are first diagnosed with relapsing-remitting MS but develop secondary-progressive MS (SPMS) after a number of years. Relapsing-remitting multiple sclerosis (RRMS) describes the initial course of 85% to 90% of individuals with MS. This subtype is characterized by unpredictable attacks (relapses) followed by periods of months to years of relative quiet (remission) with no new signs of disease activity. Deficits suffered during the attacks may either resolve or may be permanent. When deficits always resolve between attacks, this is referred to as "benign" MS.

Only a few disease-modifying treatments have been approved by regulatory agencies of different countries for RRMS. Three of them are interferons: two formulations of interferon beta-1a (trade names Avonex and Rebif) and one of interferon beta-16 (U.S. trade name Betaseron, in Europe and Japan Betaferon).

Interferons (IFNs) are inducible cytokines that have potent antiviral and antiproliferative effects. IFN-beta (IFNβ), a type I IFN, is the most commonly used treatment for RRMS. It decreases relapse rates by approximately 30%, reduces brain magnetic resonance imaging (MRI) activity, and slows progression of disability¹⁻³.

The US 2005/0064483 discloses methods for monitoring multiple sclerosis patients taking beta-IFN. The methods disclosed therein do not allow a prediction if a treatment will be successful for a patient prior to administration of IFNβ. In the specific method of claim 11, the method is for predicting a response. However, this method requires treating the sample ex vivo with IFNβ.

### Problem

A problem of the treatment of patients with IFNβ is that a considerable fraction of patients does not respond to treatment⁴. Criteria to classify patients into responders and non-responders to IFNβ are usually applied after one or two years follow-up. Hence many patients are treated with IFNβ without ultimate benefit and at high socioeconomic cost. Furthermore, patients on ineffective therapy accumulate further disability and often suffer treatment-related side effects. It is, therefore, highly desirable to identify surrogate markers that allow early identification of treatment failure or ideally even predict non-responder status. Although several markers have previously been shown to be of potential use to this end ⁵⁻⁸, to date there is no definitive biomarker that allows to predict the response to IFNβ treatment in MS.

The problem underlying the invention is to overcome the above mentioned problems and to improve the diagnostics and therapy of multiple sclerosis, especially of RRMS. Specifically, the problem is to provide an efficient and simple method for determining whether a patient will respond to IFNβ treatment.

### Invention:

The problem underlying the invention is surprisingly solved by diagnostic methods, devices, microarrays, uses and therapeutic methods of the claims.

Subject of the invention is a method for predicting the treatment response of a multiple sclerosis patient to a type I IFN, comprising the steps of
(a) determining the expression level or activity of IFN type I or of at least one marker regulated by type I IFN in a sample obtained from said patient,
(b) comparing the level or activity to a standard,
(c) predicting the treatment response, wherein a treatment with a type I IFN is predicted to be ineffective in case the patient shows an altered level or activity of the marker.

Another subject of the invention is a method for predicting the treatment response of a multiple sclerosis patient to a type I IFN, comprising the steps of
(a) determining the expression level or activity of at least one marker in a sample obtained from said patient,
(b) comparing the level or activity to a standard,
(c) predicting the treatment response, wherein a treatment with a type I IFN is predicted to be ineffective in case the patient shows an altered expression level or activity of the marker,
wherein the patient was not subjected to a type I IFN treatment prior to obtaining said sample, and/or wherein the method does not comprise a step of contacting said sample obtained from said patient with a type I IFN.

As used herein, a "standard" refers to any control sample or control value that serves as a reference in the present invention. The standard is thus a known standard. In a preferred embodiment of the invention, the control sample is isolated from patients without MS, preferably from healthy patients. The control sample is preferably from a patient who has not been treated with a type I IFN, or a patient which has been treated such a long time ago that the effects of the treatment are not observed any more. The control sample may be from an individual or from a pool. The method of the invention is advantageous, because it allows a prediction if a treatment with type I IFN will be successful, without the need to administer the type I IFN to the patient. However, the method of the invention can also be used to monitor a patient in the first months of IFNβ administration. In such a patient, it can be predicted whether a further treatment is reasonable. In the method of the invention, it is not necessary that the sample is treated with IFNβ as disclosed in US2005/0064483 in order to study a response to IFNβ by simulating a treatment with IFNβ *ex vivo*. In a preferred embodiment of the invention, the method does not comprise contacting, especially incubating the sample with IFNβ.

An altered or increased level or activity deviates significantly from the standard. A deviation is "significant" if it is unlikely that the deviation occurs in a healthy individual, for instance if the probability that it occurs in a healthy individual is less than 20, 10 or 5%.

An altered expression level or activity of the marker allows the prediction that a treatment with type I IFN will be ineffective. In contrast, an identical or a similar level or activity, which is not significantly altered, allows the prediction that a treatment type I IFN will be effective.

It is well known to the skilled person that in general, a prediction is more likely to be valid if the measured deviation from a standard is strong. Further, a prediction is more likely to be valid in case a deviation is observed for more than one marker. In a preferred embodiment, the patient shows an "altered" type I IFN response profile. A "profile" relates to a plurality of at least 2 deviations. An "altered type I IFN response profile" is a profile which significantly deviates from a standard profile. In preferred embodiments, at least the expression levels of 2, 3 5 or 10 markers are altered. In general, the prediction is more valid if the number of markers with altered expression levels is high and if the deviations are strong.

In general, the altered profile can be an increased expression, decreased expression, increased activity or decreased activity. However, as outlined in detail below it was found that for most markers a treatment with a type I IFN is predicted to be ineffective in case the patient shows an increased expression level of the marker.

The term "altered expression level" means that the relative expression is decreased or increased in one sample compared to the standard sample. Parameters for increased expression may change as necessary for a particular algorithm. For example, it is contemplated that a gene is considered increased expressed if its expression is at least 10, 20, 50 or 100% higher than the standard sample. However, the skilled person understands that a deviation of, for example, 50% can be more significant for one marker than for another, and thus for a valid prediction, it is advantageous to evaluate an expression profile.

The term "regulated by type I IFN" means, that the physiological level or activity of the marker is depending on the level or activity of the type I IFN, and changes in the activity or level of the type I IFN result in an altered level or activity of the marker.

Preferably, before taking the sample for the diagnostic assay of the invention, the patient was not subjected to a type I IFN treatment and/or the method of the invention does not comprise a step of contacting the sample obtained from the patient with a type I IFN. In a preferred embodiment, the physiological expression level or activity is determined. "Physiological" means, that the measurement, although performed in the sample *in vitro*, shall as much as possible reflect the expression level or activity in the patient. In other words, the expression level or activity is preferably determined *ex vivo*. In this context, the term "*ex vivo*" refers to measurements done in an environment outside the organism with the minimum alteration of the natural conditions before the measurement. Therefore, prior to the measurement the sample should not be manipulated in a way that the determined level or activity will not reflect the level or activity in the patient. For instance, before the measurement the sample should not be subjected to IFNs.

Preferably, the sample comprises cells or is obtained from cells. Preferred samples for the present invention are peripheral blood mononuclear cells (PBMCs), or those comprising PBMCs. PBMCs are cells in the bloodstream that have one round nucleus. PBMCs comprise lymphocytes and monocytes. PBMCs can be isolated from whole blood samples using different density gradient centrifugation procedures. Anticoagulated whole blood is layered over the separating medium. At the end of the centrifugation step, the following layers are visually observed from top to bottom: plasma/platelets, PBMCs, separating medium and erythrocytes/granulocytes. The PBMC layer is then removed and washed to get rid of some contaminants before cell type and cell viability can be confirmed. In a preferred embodiment of the invention the sample comprises monocytes or is obtained from monocytes. A monocyte is a type of leukocyte, part of the human body's immune system.

In a preferred embodiment, the sample comprises enriched or purified nucleic acids. Preferably, the sample is the total mRNA isolated from a biological sample. The term "biological sample", as used herein, refers to a sample obtained from an organism or from components (e.g., cells) of an organism. The sample may be any biological tissue or fluid or derived therefrom. Frequently the sample will be a "clinical sample" which is a sample derived from a patient. Such samples include sputum, blood, blood cells (e.g., white cells), tissue or fine needle biopsy samples, urine, peritoneal fluid, pleural fluid, cerebrospinal fluid (CSF), or cells or isolated and fractions purified therefrom. Samples may also include sections of tissues such as frozen sections taken for histological purposes.

In preferred embodiments of the invention, the marker is an mRNA, a cDNA, a gene or a protein. The term "mRNA" refers to a transcript of a gene. Transcripts are RNA including, for example, mature messenger RNA ready for translation or products of various stages of the transcript processing. Transcript processing may include splicing and degradation. Methods of isolating total mRNA are well known to those of skill in the art. For example, methods of isolation and purification of nucleic acids are described in detail in Chapter 3 of Laboratory Techniques in Biochemistry and Molecular Biology: Hybridization with Nucleic Acid Probes, Part I. Theory and Nucleic Acid Preparation, P. Tijssen, ed. Elsevier, N.Y. (1993)

The determination of the level or activity of the marker can be performed by methods known in the art. For example, gene expression data is obtained by employing an array of probes that hybridize to several, and even thousands or more different RNA transcripts. Such arrays are often classified as microarrays or macroarrays, and this classification depends on the size of each position on the array. However, the determination can also be performed without an array by other known techniques. For example, the level of nucleic acids can also be determined by PCR, preferably by real time PCR (RT-PCR), or by an RNA blot (Northern blot). The level of phosphorylation of a marker, such as STAT1, can be determined by flow cytometry. The level of cell surface markers can be determined by immunostaining. The expression level of proteins can be determined by western blot, SDS-PAGE or 2D-electrophoresis. The activity of proteins can be determined by known assays, for example measurement of the conversion of substrates.

In a preferred embodiment of the invention, in step (a) the expression level of at least one mRNA is determined and in step (c) a treatment with a type I IFN is predicted to be ineffective in case the patient shows an increased expression level of the at least one mRNA.

In one embodiment, the present invention provides a method wherein nucleic acid probes are immobilized on or in a solid or semisolid support in an organized array. Oligonucleotides can be bound to a support by a variety of processes, including lithography, and where the support is solid, it is common in the art to refer to such an array as a "chip" or "biochip".

One embodiment of the invention involves monitoring gene expression by (1) providing a pool of target nucleic acids comprising RNA transcript(s) of one or more target gene(s), or nucleic acids derived from the RNA transcript(s); (2) hybridizing the nucleic acid sample to a array of probes (including control probes); and (3) detecting the hybridized nucleic acids and calculating a relative expression (transcription) level.

Type I IFNs bind to a specific cell surface receptor complex known as the IFNα receptor (IFNAR) that consists of IFNAR1 and IFNAR2 chains. The type I interferons present in humans are IFNα, IFNβ and IFNω. In a preferred embodiment of the invention, the type I IFN is INFβ.

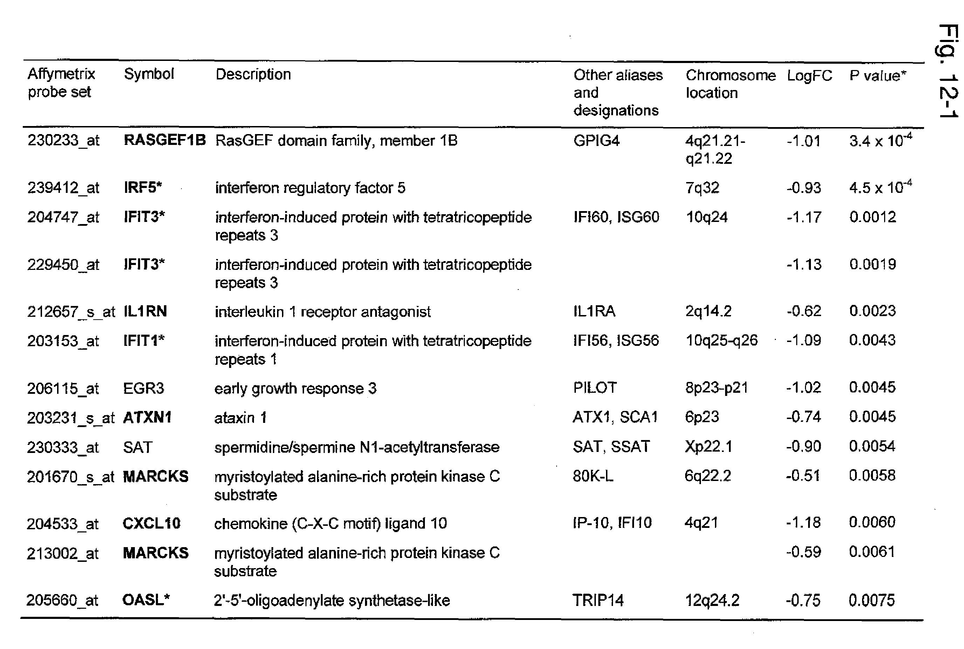

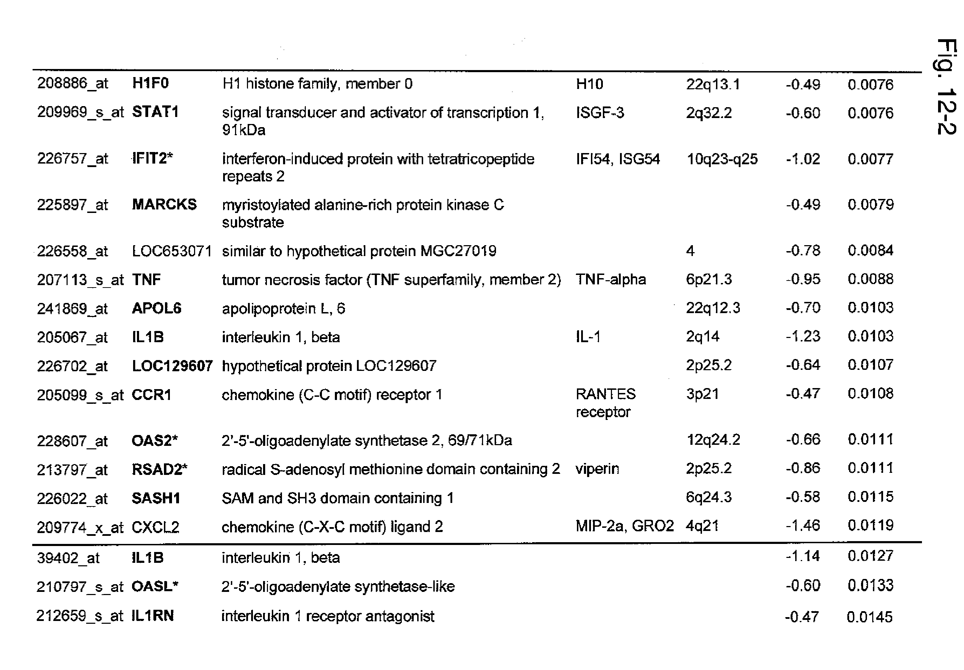

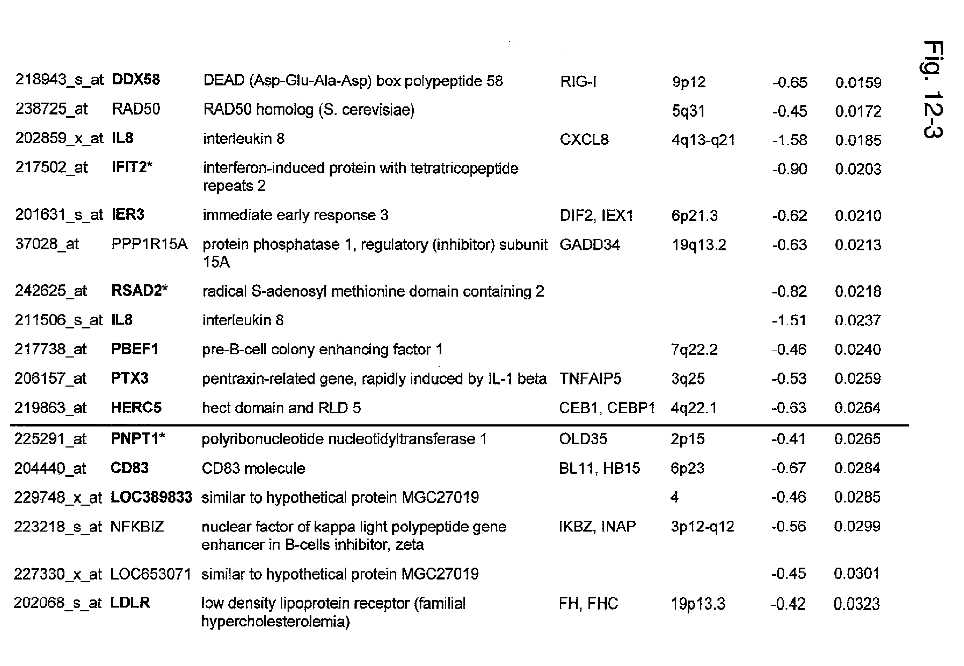

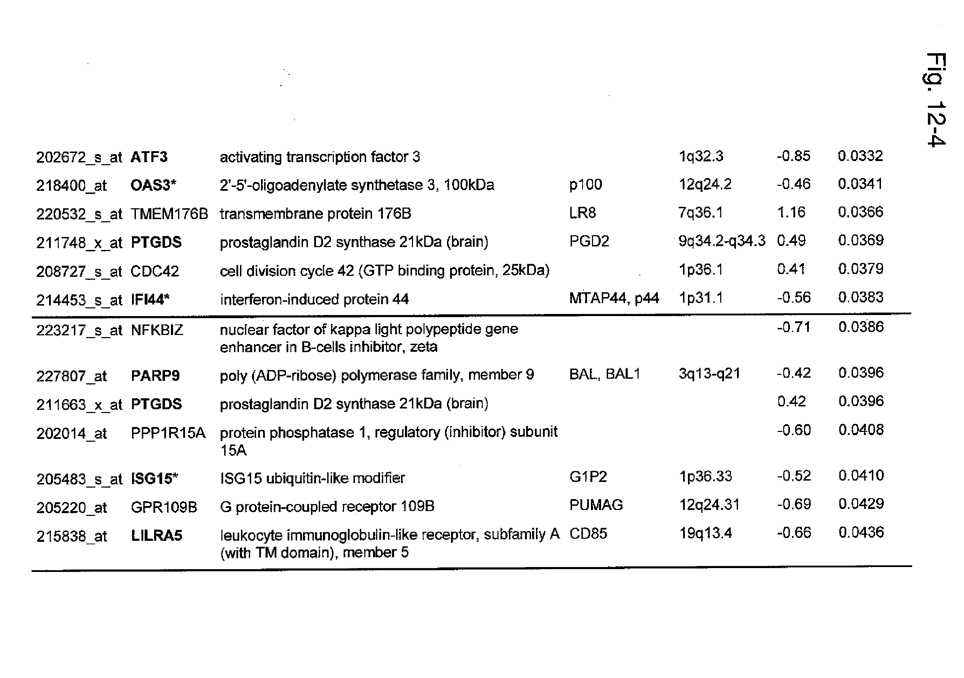

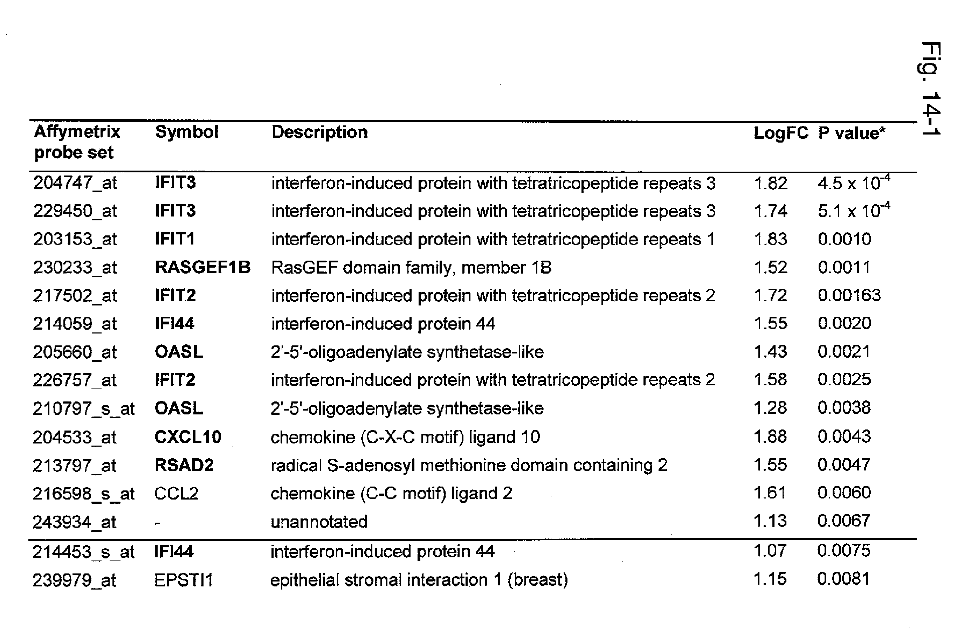

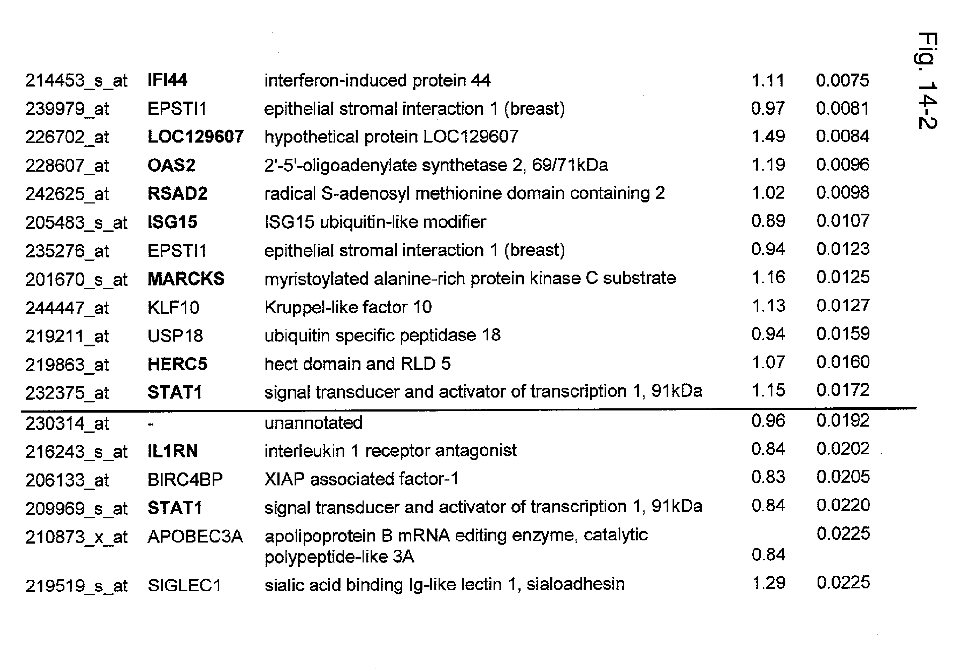

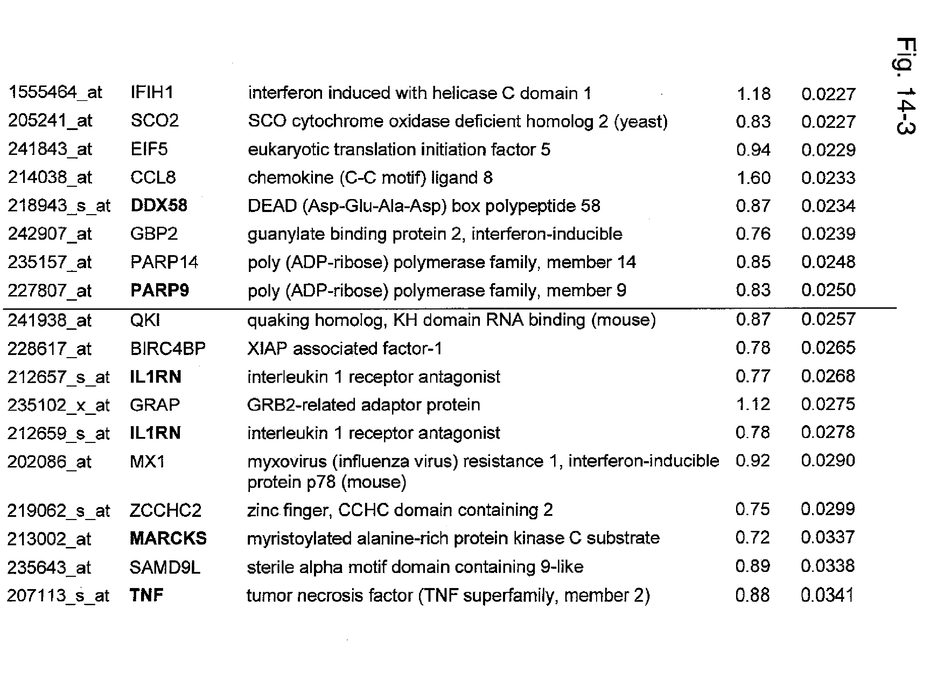

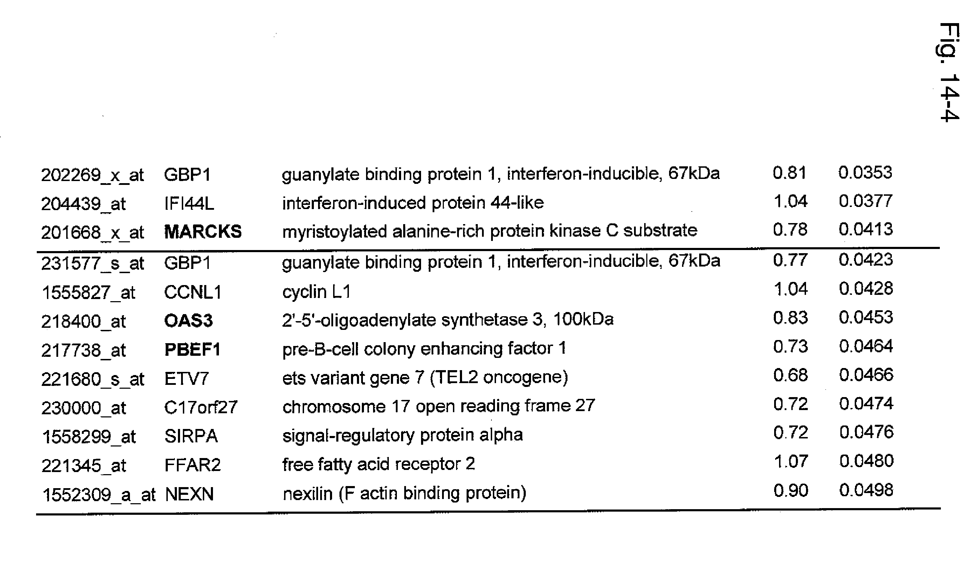

In a preferred embodiment of the invention, in step (a) the expression level of at least one marker is determined, the marker being selected from the group consisting of IFIT3, IFIT1, IFIT2, RASGEF1B, IFI44, FADS1, OASL and MARCKS,
wherein the treatment is predicted to be ineffective in case the level is increased. The specific denotation of the markers is shown in figure 12 and 14.

In a preferred embodiment of the invention, in step (a) the expression level of at least one marker is determined, selected from the group consisting of RASGEF1B, IRF5, IFIT3, IL1RN, IFIT1, ATXN1, MARCKS, CXCL10, OASL, H1F0, STAT1, IFIT2, TNF, APOL6, IL1B, LOC129607, CCR1, OAS2, RSAD2, SASH1, DDX58, IL8, IER3, PBEF1, PTX3, HERC5, PNPT1, CD83, LOC389833, LDLR, ATF3, OAS3, PTGDS, IFI44, PARP9, ISG15, LILRA5, EGR3, SAT, LOC653071, CXCL2, RAD50, PPP1R15A, NFKBIZ, LOC653071, TMEM176B, CDC42, NFKBIZ, PPP1R15A and GPR109B.

In preferred embodiments of the invention, in step (a) at least one of the following is determined:
(a1) type I IFN secretion of monocytes upon innate immune stimuli via TLR4, wherein the treatment with a type I IFN is predicted to be ineffective in case the secretion is increased,
(a2) serum type I IFN bioactivity, wherein the treatment with a type I IFN is predicted to be ineffective in case the secretion is increased,
(a3) an activation status of myeloid DCs, wherein the treatment with a type I IFN is predicted to be ineffective in case the status is elevated,
(a4) the T helper cell response towards Th1, wherein the treatment with a type I IFN is predicted to be ineffective in case the cell response is preferentially skewed,
(a5) the level of phosphorylated STAT1, wherein the treatment with a type I IFN is predicted to be ineffective in case the level is increased.

In a preferred embodiment of the invention the treatment is predicted to be ineffective if the level or activity of the marker is increased at least by 10, 20, 50 or 100%.

In specific embodiments of the invention, an altered level of expression, which for instance can be performed according to the methods described below in the methods section, means a p-value at baseline of <0.05, preferably <0.25, more preferably <0.1.

Another subject of the invention is a method for treating multiple sclerosis in a patient showing an altered type I IFN response profile, comprising the step of administering a substance for neutralizing type I IFNs. Preferably, the substance is an antibody or an interferon inhibitor.

In a preferred embodiment of the invention, the determination of the level or activity of the marker is performed in an automated manner, for instance by a robot.

Robots for analyzing microarrays or performing measurements for high numbers of samples, for instance with 96-well plates, are known in the art.

Another subject of the invention is a micorarray for predicting the treatment response of a multiple sclerosis patient to a type I IFN, wherein the microarray comprises not more than 200, preferably not more than 100, 50 or 30 markers and comprises at least 3, 5 or 10 markers for determining an altered type I IFN response profile. In preferred embodiments, the arrays comprise 5 to 100, 5 to 50 or 10 to 30 markers known to be associated with a type I IFN signature. In a further preferred embodiment, the microarray comprises only specific markers selected from those disclosed above. Such a microarray would be a specific microarray for assessing a type I IFN signature. In addition, the microarray could comprise at least one marker not affected by a type I IFN as a control. Microarray analysis is a useful method for a highly efficient analysis of gene expression profiles. Preferably, the microarray is an ordered array of genes immobilized on a planar substrate that allows the specific binding of labeled nucleic acids. Microarray technologies which can be used for analyzing gene expression profiles involve depositing nucleic acids on a solid platform in a set pattern, and hybridizing a solution of complementary nucleic acids to the nucleic acid targets. Microarray technology has been applied increasingly due to its capability to illustrate changes in the cellular behavior on a genomic level.

In a preferred embodiment of the invention, the markers are nucleic acid probes attached to a solid surface. In a specific embodiment, the solid surface is nitrocellulose or glass. Preferably, the solid support may be glass, plastic (e.g., polypropylene, nylon), polyacrylamide or nitrocellulose. A preferred method for attaching the nucleic acids to a surface is printing on glass plates, Another preferred method for making microarrays is by making high-density oligonucleotide arrays. Techniques are known for producing arrays containing thousands of oligonucleotides complementary to defined sequences, at defined locations on a surface using photolithographic techniques for synthesis in situ.

If the level of a plurality of markers is studied, an "expression profile" or "gene expression profile" is generated. The profile indicates the relative expression or relative abundance of any particular transcript. The compilation of the expression levels of all of the mRNA transcripts sampled at any given time point in any given sample comprises the gene expression profile.

In a preferred embodiment, the micorarray for predicting the treatment response of a multiple sclerosis patient to a type I IFN comprises at least 3 markers selected from the group consisting of RASGEF1B, IRF5, IFIT3, IL1RN, IFIT1, ATXN1, MARCKS, CXCL10, OASL, H1F0, STAT1, IFIT2, TNF, APOL6, IL1B, LOC129607, CCR1, OAS2, RSAD2, SASH1, DDX58, IL8, IER3, PBEF1, PTX3, HERC5, PNPT1, CD83, LOC389833, LDLR, ATF3, OAS3, PTGDS, IFI44, PARP9, ISG15, LILRA5, EGR3, SAT, LOC653071, CXCL2, RAD50, PPP1R15A, NFKBIZ, LOC653071, TMEM176B, CDC42, NFKBIZ, PPP1R15A and GPR109B.

Subject of the invention is also the use of the microarray of the invention for predicting the treatment response of a multiple sclerosis patient to a type I IFN.

Subject of the invention is also a diagnostic kit comprising a microarray or the marker of the invention. Such a kit comprises the markers, which may be arranged on an array or another device for diagnosis. The kit preferably comprises solutions for carrying out the sample preparation and/or the determination of the marker expression level or activity. Additionally, the kit may comprise the necessary devices, such as test tubes, or chemicals, for instance for staining probes.

The inventors performed genome-wide expression profiling in MS patients clinically classified as responders and non-responders. Before treatment, IFNβ responders and non-responders are characterized by differential expression of type I IFN-induced genes. Upon treatment the expression of these genes remains unaltered in non-responders, but is strongly upregulated in responders. Functional experiments showed a selective increase in phosphorylated STAT1 levels and IFN receptor 1 (IFNAR1) expression in monocytes of non-responders at baseline. A poor clinical outcome was furthermore associated with increased serum type I IFN bioactivity and elevated activation status of myeloid dendritic cells. These findings indicate that perturbations of the type I IFN pathway in monocytes are related to a poor outcome in MS, and type I IFN-regulated genes may be used as response markers in IFNβ treatment in MS.

The invention provides biomarkers that are associated with or even predictive of the response to IFNβ treatment in RRMS patients by genome-wide gene expression profiling in PBMC.

In the study underlying the invention, IFNβ responders and non-responders respectively were characterized by very stringent and conservative criteria that had previously been applied²¹. IFNβ responders had to be free of relapses and disease progression for at least 24 months, and on the other hand non-responders had to show both relapses and sustained progression. In a preferred embodiment, the method of the invention results in a prediction if the patient is a responder or a non-responder within the definition given herein. Preferably, the non-responder shows a type I IFN signature.

IFNβ non-responders express a number of type I IFN-regulated genes at elevated levels already at baseline, i.e. prior to any exogenously administered IFNβ. Furthermore, upon treatment initiation these differentially expressed genes are induced in responders, but their regulation appears blunted in non-responders. In addition, the genes *IFIT1-3* and a few others, which are selectively induced by type I IFNs, were the best predictors of the therapeutic outcome after 24 months of treatment. When dissecting this type I IFN signature further, IFNβ non-responders are characterized by elevated expression of IFNAR1 in monocytes, by increased type I IFN secretion of monocytes upon innate immune stimuli via TLR4, by increased serum type I IFN bioactivity, an elevated activation status of myeloid DCs, and, consistent with this, a preferential skewing of the T helper cell response towards Th1. In a preferred embodiment of the invention, the measurement of the expression level in step (a) is obtained at baseline. Thus a confounding role of neutralizing antibodies (NABs) against IFN can be excluded.

A type I gene expression IFN signature has been reported in several autoimmune disorders such as rheumatoid arthritis, systemic lupus erythematosus, dermatomy-ositis, systemic sclerosis, and Sjögren's syndrome. In MS, a recent study using gene expression microarrays observed the presence of a selective upregulation of the type I IFN signalling pathway in a subgroup of patients with RRMS¹⁶. This shared type I IFN signature may suggest the presence of common etiological factors and pathogenetic pathways operating in these autoimmune disorders. In the present invention, it was observed that the type I IFN signaling pathway is implicated in the response to IFNβ treatment. This pathway seems to be selectively altered in the monocyte lineage in non-responders, as the increase in p-STAT1 levels and IFNAR1 expression was observed only in this cell population. Furthermore, the increased serum production of IFNα after TLR4 stimulation was most likely mediated by monocytes.

Monocytes are among the main components of inflammatory infiltrates in MS brains, and infiltrating myeloid DCs as well as microglia are thought to drive brain inflammation via activation and expansion of pathogenic T cells. A deficient expression of negative regulators of the type I IFN-induced Jak/STAT signalling pathway or reactivation of latent viral infections by EBV and HHV-6 as the causes of the overexpression of type I IFN-responsive genes in monocytes of IFNβ non-responders was excluded by control experiments. Furthermore, no indication was found of a broad activation of monocytes or other PBMC populations, as expression of activation markers was similar in responders and non-responders to treatment. Instead, the results point to an increased or prolonged signalling through the IFNAR mediated by high levels of type I IFNs, as indicated by the elevated type I IFN bioactivity and the increased production of IFNα after TLR4 stimulation observed in non-responders.

When considering the causes of the IFNβ unresponsiveness, it appears that the IFNβ non-responders probably represent a pathogenetically different phenotype of MS with clinically more severe course, altered monocyte function, and an activated innate immune system. The latter notion is supported by the upregulation of several chemokines/receptors (CXCL10, interleukin-8, CCR1) and monocyte-derived monokines (IL-1β, TNFα) and -surface molecules (CD85) in non-responders. In these patients the administration of exogenous IFNβ failed to induce the expression of a number of type I-regulated genes, which are strongly upregulated in responders. Currently, it cannot be distinguished whether the presence of relapses and progression of neurological disability in non-responders during the two years of treatment is due to lack of response to IFNβ or more active disease, or a combination of both. However, according to the invention a subgroup of patients is identified, who on the one hand have a more activated and less inducible type I IFN pathway; and these patients fail to respond to IFNβ treatment. On the other hand, in these patients that were labelled as non-responders, for yet unknown reasons, the type I IFN pathway is dysregulated in monocytes, which may contribute to more active disease. The biological relevance of this observation is further underscored by a recent study that documents that conditional genetic knockout of IFNAR1 in monocytes, but not in T cells, B cells or CNS cells, leads to enhanced disease severity in the animal model of MS, experimental autoimmune encephalomyelitis.

In the context of a type I IFN signature, type I IFNs may not be beneficial for MS, as they are known to activate DC²⁹, enhance humoral immunity³⁰, and favor Th1 immune responses³¹. In fact, at baseline in non-responders myeloid DC are more activated - and this DC subtype is considered particularly relevant in MS pathogenesis²⁷, serum induces differentiation of monocytes into DC, and activated PBMC are characterized by a skewing towards Th1 type immune responses, which is mediated by type I IFN-activated STAT4¹⁸⁻²⁰. These findings are likely to be mediated by higher levels of type I IFNs, possibly IFNα, in non-responders.

Based on these observations, it appears counterintuitive to administer IFNβ, a type I IFN, which in many aspects has overlapping functions with IFNα, to patients with overexpression of type I IFN-responsive genes under basal conditions and relative unresponsiveness of the type I IFN pathway to the effects of IFNβ.

Therefore, one embodiment of the invention is a method of treating non-responder MS patients by neutralization of type I IFNs. Such an approach has been proposed for other autoimmune disorders characterized by a type I IFN signature.

Besides its well documented antiviral function, a broad range of biological activities and immunomodulatory properties has been documented for IFNβ. According to the state of the art, it was not clear whether treatment efficacy is due primarily to a single or rather a combination of different causes. Furthermore, it was conceivable that incomplete or complete treatment failure occurs due to different biological effects in individual patients. As examples, treatment failure could be related to lack of inhibition of matrix metalloproteinases, to absence of soluble adhesion molecule upregulation or to formation of neutralizing anti-IFNβ antibodies. Given the central role of type I IFNs play in protective host responses, the inventors considered it unlikely that MS patients, who are not responding to therapeutic IFNβ, show major deficiencies in the type I IFN function or signaling. Instead, it was anticipated that alterations in the response to IFNβ are subtle and affect multiple biological pathways. Therefore, in a study genome-wide gene expression profiling by oligonucleotide microarrays was employed to identify expressed genes or cellular pathways associated with the clinical response to IFNβ treatment in a cohort of RRMS patients with a clinical follow-up of at least 2 years on therapy.

The invention is based on the PBMC from IFNβ non-responders are characterized by a type I IFN signature and a relative unresponsiveness to treatment with IFNβ. In addition, this pathway is selectively altered in monocytes, possibly in the context of increased production of type I IFNs in non-responders. The invention thus defines response markers to identify patients (non-responders), who will not benefit from IFNβ either before treatment is initiated or in the first months of IFNβ administration. The altered type I IFN response profile in monocytes in this subgroup of patients offers new leads towards the investigation of pathogenetic mechanisms in MS and has clear implications for clinical management of MS patients as well.

### Examples

### Methods:

### Study design and clinical assessment

This is a prospective study of RRMS patients receiving treatment with IFNβ at our centre (HUVH). Patients were included in a follow-up protocol collecting basal and longitudinal clinical data, including number of relapses and EDSS scores, as previously described²¹. The study was approved by the local ethics committee, and all patients gave their informed consent.

### Definition of response to IFNβ therapy

Clinical criteria of response to IFNβ were applied after two years of treatment. Patients were classified as responders when there was no increase in the EDSS score and no relapses during the follow-up period. Patients were labeled as non-responders when if they experienced one or more relapses and an increase of at least 1 point in the EDSS score that persisted for a minimum of two consecutive visits separated by a 6-month interval²¹. These stringent clinical criteria were applied in order to discriminate clearly between responders and non-responders and avoid patients having overlapping clinical responses to treatment. Patients with intermediate phenotypes of responses, i.e., presence of relapses and increase of less than 1 point in the EDSS score were not included in the study.

### Patients

Forty-seven RRMS patients were included in the study. There were 29 (61.7%) patients classified as responders and 18 (38.3%) classified as non-responders.
None of these patients had ever received treatment with IFNβ or other immunosuppressive therapy before study entry. No patient had exacerbations or received treatment with corticosteroids during the month before study entry.

### RNA isolation and expression profiling using microarrays

Total RNA was extracted from PBMC using an RNeasy kit (Qiagen, Santa Clarita, USA) according to the manufacturer's recommendations. Extracted total RNA was used to synthesize double stranded cDNA using the One Cycle cDNA Synthesis Kit (Affymetrix, Inc.). Biotin-labeled antisense cRNA was obtained using the same kit starting with 5 µg of total RNAs and the oligo dT primer 5' GGCCAGTGA-ATTGTAATACGACTCACTATAGGGAGGCGG-(dT)24. cRNAs were purified using the columns from the GeneChip® Sample Cleanup Module (Affymetrix, Inc.) and then 20 µg of cRNAs were fragmented at 94°C for 30 minutes in 40 µl of 40 mM Tris-acetate, pH 8.1, 100 mM KOAc, 30 mM Mg(OAc)2. The fragmented samples were added to a hybridization cocktail containing Control oligonucleotide B2 (50 pM) and Eukaryotic Hybridization controls (BioB, BioC, BioD, cre) at 1.5, 5, 25 and 100 pM final concentration respectively from the GeneChip Eukaryotic Hybridization Control Kit (Affymetrix, Inc.), herring sperm DNA (0.1 mg/ml) and acetylated BSA (0.5 mg/ml). Probe array was equilibrated to RT and prehybridized with 1x hybridization buffer (100mM MES, 1M [Na+], 20mM EDTA, 0.01% Tween 20) at 45°C for 10 minutes with rotation. Two-hundred µl of the mixture were used for hybridization to the Human Genome U133A Plus 2.0 arrays (Affymetrix Genechip® Array; Santa clara, CA) at 45°C for 16 hours with rotation. GeneChips were washed and marked with streptavidin phycoerythrin using the protocol EukGE-WS2-v5 provided by Affymetrix. Once washed and streptavidin phycoerythrin-marked the GeneChips were scanned in an Agilent G3000 GeneArray Scanner. The Human Genome U133A Plus 2.0 arrays allows a complete coverage of the Human Genome U133 Set plus 6,500 additional genes for analysis of over 47,000 transcripts.

### Statistical analysis of microarray data

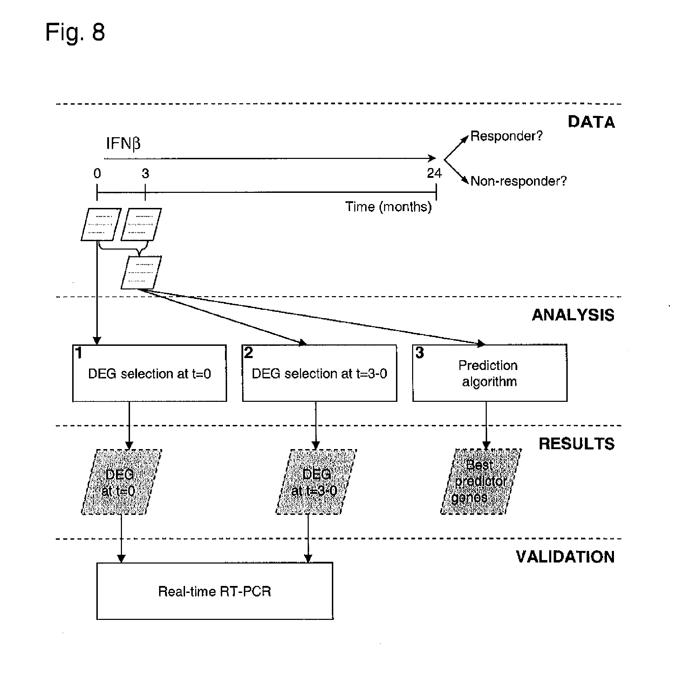

A schematic flow chart summarizing the main steps performed in the analysis of microarray data is represented in **Fig. 8**. Analysis of the expression data was carried out by using the Bioconductor packages for the R programming environment. All chips were one by one explored and checked for quality. After that, data obtained from .CEL files were preprocessed using the RMA method³³. These preprocessed values were the basis for all the analysis. To avoid genes with low signal and little variation in their expression among all patients, a two step non-specific filtering was carried out: first, genes not reaching a minimum mean signal in all groups were left out of the analysis; second, from the selected genes, only those having variability (standard deviation was chosen as the comparison parameter) between all arrays greater than a certain percentile were kept for further analysis. Both thresholds were chosen after studying the behavior of the expression data for each step of the analysis. The selection of differentially expressed genes between responders and non-responders at baseline and in the first 3 months of treatment with IFNβ was based on a linear model analysis with empirical Bayes moderation of the variance estimates following the methodology developed by Smyth *et al*. (2003)³⁴. In order to deal with the multiple testing issues derived from the fact that many tests are performed simultaneously, as a linear model is generated for each gene, p-values were adjusted to obtain strong control over the false discovery rate (FDR), which is defined as the expected proportion of Type 1 or false positives among the rejected hypothesis using Benjamini and Hochberg's method (1995)³⁵.

Studies on the prediction of response to treatment were performed based on changes in gene expression induced by IFNβ in the first three months of treatment. Thus, the difference in the expression levels between three months and baseline was computed for each gene in responders and non-responders. A prediction algorithm was generated building and comparing several predictors in an iterated process which was cross-validated in order to avoid well known problems such as overfitting or selection bias³⁶. In this way, data were partitioned randomly in ten balanced sets and successively one of the partitions was considered as validation set whereas the other nine parts were considered as training set. For each training set predictors were constructed and applied to the validation set. This procedure was repeated several times with an increasing subset of genes ranging from 2 to a hundred. In this process, known as feature selection, we considered a maximum of 100 genes, given that our purpose was to find a small list of genes that could help to predict response to treatment. Genes to be included in the predictors were selected using the F test known as "between to within sums-of-squares ratio"³⁷. The following candidate predictors were evaluated: Diagonal Linear Discriminant Analysis (DLDA), k-Nearest Neighbor (kNN) with 1, 3, 5, and 7 neighbors, Support Vector Machine (SVM) with several parameters, Nearest Shrunken Centroids, and Random Forest. The selection of best predictor genes was done taking the subset with the number of genes that had associated the smallest estimated misclassification error. We then calculated the definitive list by using all data and selecting again the best number of genes founded in the feature selection step. The identification of relevant pathways in differentially expressed genes between responders and non-responders was performed with the package "sigPathway" implemented in R³⁸.

### Validation of microarray data with real-time quantitative reverse transcription polymerase chain reaction (RT-PCR)

Real-time RT-PCR was used to validate differential expressed genes obtained with microarrays at baseline and in the first months of treatment. Total RNA was taken from the same samples that had been used for the microarrays. cDNA was synthesized from 150 ng of total RNA using the High Capacity cDNA Archive Kit (Applied Biosystems, Foster City, CA, U.S.A), combined with TaqMan® Universal PCR Master Mix and loaded in duplicates into Taqman® Low-density Arrays containing TaqMan® Gene Expression Assays specific for the genes selected to validate microarray data. The housekeeping gene 18S rRNA was used as an endogenous control. Taqman arrays were run on the ABI PRISM® 7900HT system (Applied Biosystems). The threshold cycle (C_{T}) value for each reaction, and the relative level of gene expression for each sample were calculated using the 2^{-ΔΔCT} method³⁹. Briefly, 18S rRNA was employed for the normalization of the quantity of RNA used. Its C_{T} value was subtracted from that of the specific genes to obtain a ΔCT value. The difference (ΔΔCT) between the ΔCT values obtained for the responders (calibrators) and the ΔCT values for the non-responders was determined. The relative quantitative value was then expressed as 2^{-ΔΔCT}, representing the fold change in gene expression normalized to the endogenous control and relative to the calibrators.

### Detection of basal and IFNβ-induced STAT1 phosphorylation by flow cytometry in different PBMC populations

The methodology for the detection of STAT1 phosphorylation by flow cytometry was adapted from that described by Lesinski and Dhodapkar^{40,41}. PBMC obtained at baseline were treated with and without recombinant IFNβ-1a (10,000 IU/ml) in serum-free conditions at 37°C for 20 minutes. Cells were fixed immediately and permeabilized with 90% methanol for 30 minutes on ice. Cells were washed an stained with a PE-coupled mouse anti-human phospho STAT1 antibody (BD Biosciences, San Jose, CA, U.S.A) and an IgG1-PE isotype control antibody as well as with directly coupled monoclonal antibodies specific for CD3, CD14, and CD 19 (all BD Biosciences) as per the manufacturer's protocol. Data were acquired on a BD-LSRII flow cytometer and analyzed using FlowJo software (TreeStar, Ashland, OR, U.S.A). The geometric mean fluorescence intensity of the IgG1-PE isotype control antibody was subtracted from the geometric mean fluorescence intensity of the phospho STAT1 antibody.

### mRNA levels of negative regulators of type I IFN-induced Jak/STAT signalling pathway

The mRNA levels of protein inhibitor of activated STAT1 (*PIAS1*) and suppressors of cytokine signalling 1 (*SOCS1*) and 3 (*SOCS3*) were determined by real time RT-PCR using TaqMan probes and 18S rRNA as endogenous control in a ABI Prism 7000 Sequence Detection system (Applied Biosystems). Relative quantification was performed using the 2^{-ΔΔCT} method, as described before.

### Cell surface immunostaining to detect expression of activation markers and interferon alpha receptors 1 (IFNAR1) and 2 (IFNAR2) in different cell populations

The following monoclonal antibodies were obtained from Pharmingen (San Diego, CA, U.S.A): fluorescein isothiocyanate (FITC)-conjugated mouse anti-human CD64, CD86, CD38, phycoerythrin (PE)-conjugated mouse anti-human CD80, HLA-ABC, CD25, CD83, CD14, allophycocyanin (APC)-conjugated mouse anti-human CD14, CD19, CD11c, and allophycocyanin-Cyanine 7 (APC-Cy7)-conjugated mouse anti-human CD4. Peridinine chlorophyll protein (PerCP)-conjugated mouse anti-human CD3, HLA-DR, and a cocktail of FITC-labeled anti-CD3, CD14, CD16, CD 19, CD20, and CD56 (lineage cocktail) were obtained from Becton Dickinson (Mountain View, CA, U.S.A). PE-conjugated mouse anti-human CD86 was obtained from Caltag Laboratories (Burlingame, CA, U.S.A). FITC-conjugated mouse anti-human IFNAR1 and IFNAR2 were purchased from R&D Systems (Gaithersburg, MD, U.S.A) and PBL InterferonSource (Piscata-way, NJ, U.S.A.) respectively. FITC-conjugated mouse IgG1, IgG2a, PE-conjugated mouse IgG1, APC-conjugated mouse IgG1, APC-Cy7-conjugated mouse IgG1, were obtained from Pharmingen. PerCP-conjugated mouse IgG1 and IgG2a were purchased from Becton Dickinson. To determine expression of activation markers at baseline, PBMC were stained with monoclonal antibodies against CD14, CD64, CD80, CD83, CD86, HLA-ABC, and HLA-DR for monocytes, CD19 and CD38 for B cells, CD3, CD4, and CD25 for T cells, CD11c, HLA-DR, CD80, CD83, CD86, and lineage cocktail for dendritic cells (DC), or the corresponding isotype controls. Cells were analyzed using a dual laser FACSCanto (Becton Dickinson) flow cytometer equipped with FACSDiva software. Lymphocytes and monocytes were gated based on forward and side light scatter properties. To analyze dendritic cells, cells were first gated based on forward and side light scatter properties. Myeloid DC were identified as lineage⁻HLADR⁺CD11C⁺ cells, whereas plasmacytoid DC were lineage⁻HLADR⁺CD11c⁻. Results are presented as percentage of positive cells and as mean fluorescence intensity. The expression of the surface IFNAR1 and IFNAR2 was determined at baseline in T cells, B cells, monocytes, myeloid DC, and plasmacytoid DC. Surface immunostaining and data analysis were performed as described above.

### TLR agonist stimulation and interferon quantification

Production of type I and II IFNs was examined at baseline in responders and non-responders after TLR agonist stimulation. 2x10⁵ PBMC per well were seeded in 96 well plates in 10% human AB-serum and stimulated with TLR3 (Poly(I:C); 25 µg/ml), TLR4 (LPS; 1 µg/ml), TLR7 (3M-13; 1 µg/ml), TLR8 (3M-2; 1 µg/ml), and TLR9 (CpG-C; 500mM) agonists in duplicates for 48 hours. Supernatants were analyzed for type I (IFNα2) and type II (IFNγ) interferon concentrations using a standard ELISA according to the manufacturer's recommendations (Mab-tech, Cincinnati, OH, U.S.A).

### Quantification of type I IFN bioactivity

Type I IFN bioactivity was determined in serum samples at baseline using the iLite™ Human Interferon Alpha Kit (Neutekbio, Galway, Ireland) according to the manufacturer's recommendations. This bioassay uses a stable transfected cell line derived from a pro-monocytic human cell line and detects the luciferase generated bioluminescence intensity, which is proportional to the amount of type I interferon activity (IU/ml) in the sample. A standard curve with different dilutions of IFNα-2b was used for quantification of type IFN bioactivity.

### Epstein-Barr virus (EBV) and human herpesvirus 6 (HHV-6) serologies and viral genome detection

EBV and HHV-6 replication was evaluated at baseline in the study patients. IgG and IgM serologies for EBV were performed using standard tittered immunofluorescence methods for antibodies to viral capsid antigen (VCA). HHV-6 antibodies (IgG and IgM) were determined by a commercially available ELISA (Panbio, Brisbane, Australia). In EBV and HHV-6 seropositive patients, detection of viral genome was performed by PCR in a SmartCycler II Real Time PCR System (Cepheid Inc., Sunnyvale, CA, U.S.A). Briefly, DNA was isolated from 1 x 10⁶ PBMC by means of the NucliSens® easyMAG™ automatic system (Biomerieux, Boxtel, The Netherlands). A quantitative PCR assay was used for quantification of EBV DNA viral genome load. The Artus® EBV RG PCR Kit (Qiagen, Hamburg, Germany) was utilized for specific amplification of the EBV genome following the manufacturer's recommendations. Detection of HHV-6 genome was carried out with a non-commercial qualitative PCR assay using primers and TaqMan probes as previously described⁴².

### Induction of monocyte differentiation into dendritic cells

CD14+ monocytes were purified from freshly isolated PBMC of healthy controls by negative selection using the Monocyte Isolation Kit II (Miltenyi Biotec GmbH, Bergisch Gladbach, Germany) according to the manufacturer protocol. Purity of isolated CD 14+ monocytes was >90% in all separations. Purified monocytes were cultured for 3 days (1×10⁶ cells/well) with culture media containing 25% of serum obtained at baseline from responders, non-responders, or healthy donors. Monocytes cultured with IFNα-2b (100 IU/ml) or with 25% of autologous serum were used as positive and negative controls respectively. On day 3, cells were stained for flow cytometry with monoclonal antibodies against HLA-DR, CD80, CD86, and CD83. Mean fluorescence intensity for each marker was compared between groups.

### Cytokine production in cell culture supernatants

1x10⁶ PBMC obtained at baseline were stimulated with 50 ng/ml of PMA and 1 µg/ml of ionomycin (both from Sigma, St. Louis, MO, U.S.A) for 24 hours. After stimulation, cells were centrifuged and supernatants collected and stored at -80 until used. Levels of IL-17 in the cell culture supernatants were quantified in duplicates by sandwich enzyme-linked immunosorbent assay (ELISA) using a commercially available kit (Quantikine^{R} for human IL-17, R&D Systems) according to the manufacturer's recommendations. Levels of IL-10, IFNγ and IL-4 were quantified in duplicates using custom Cytometric Bead Arrays (CBA, Becton Dickinson) according to the manufacturer's recommendations.

### Presence of neutralizing antibodies

The presence of neutralizing antibodies to IFNβ in serum samples was determined in all patients at baseline and after 12 and 24 months of treatment by means of the myxovirus A (MxA) induction bioassay, as previously described⁴³. Titers of ≥20 neutralizing units at 12 or 24 months were considered to be a positive test result.

### Results

### Analysis of differentially expressed genes at baseline reveals a type I IFN gene expression signature in non-responders

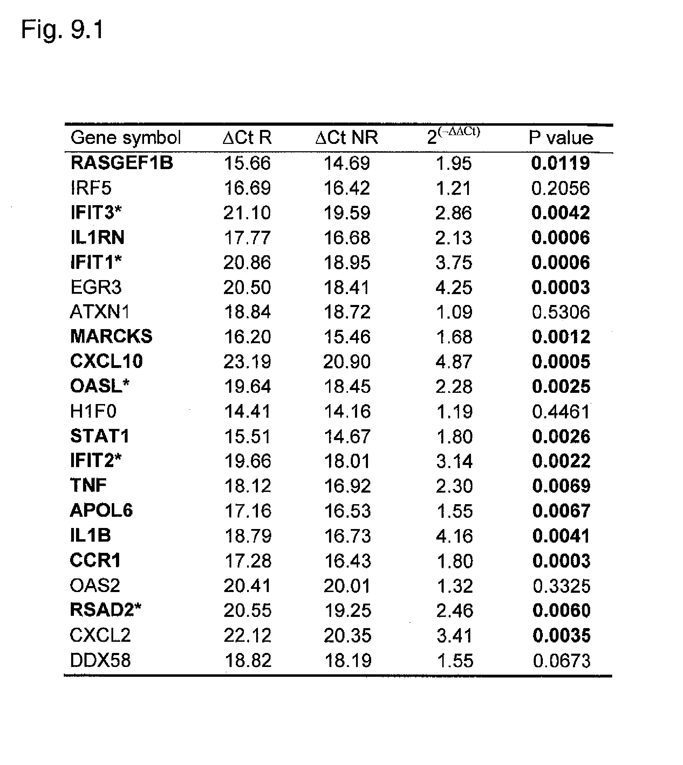

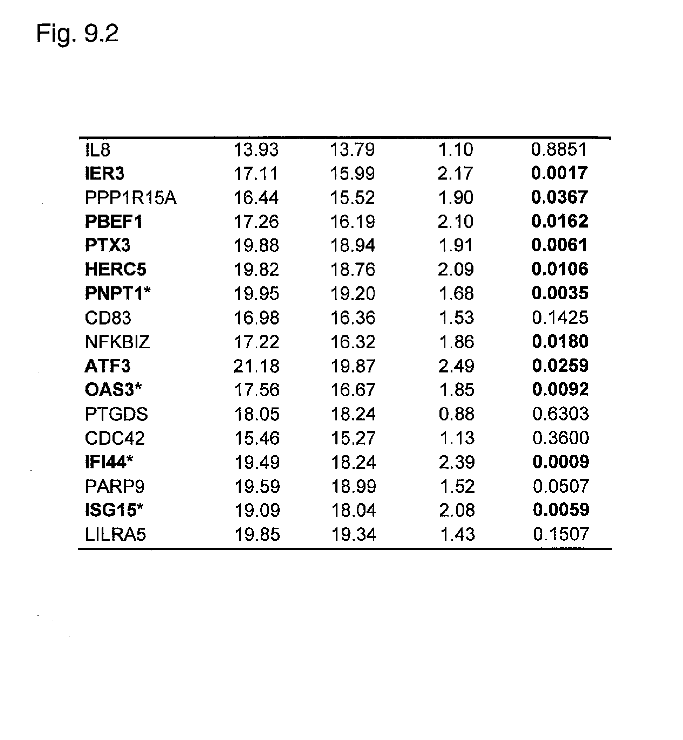

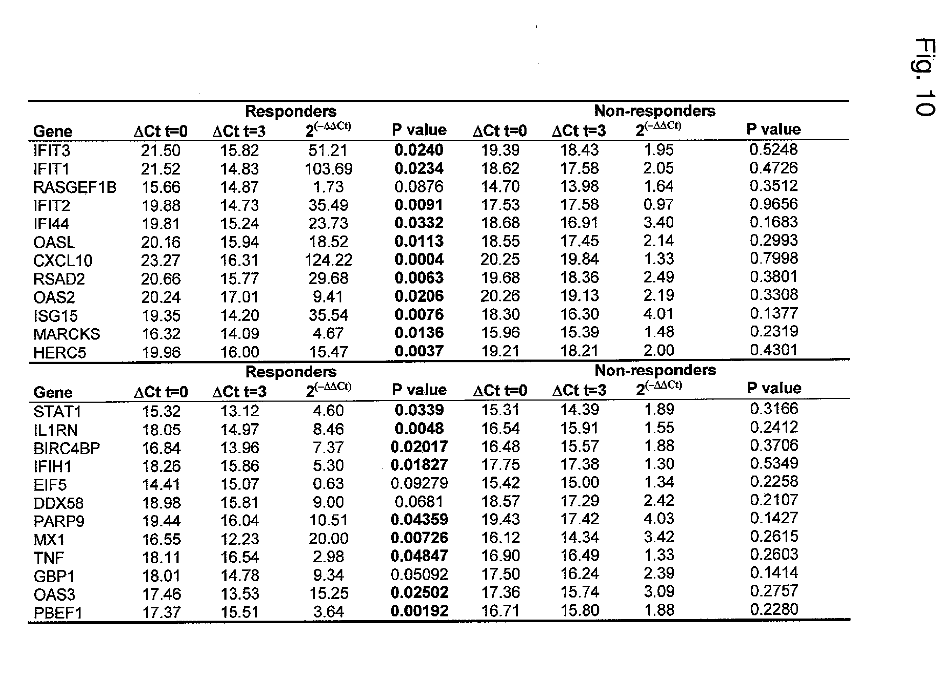

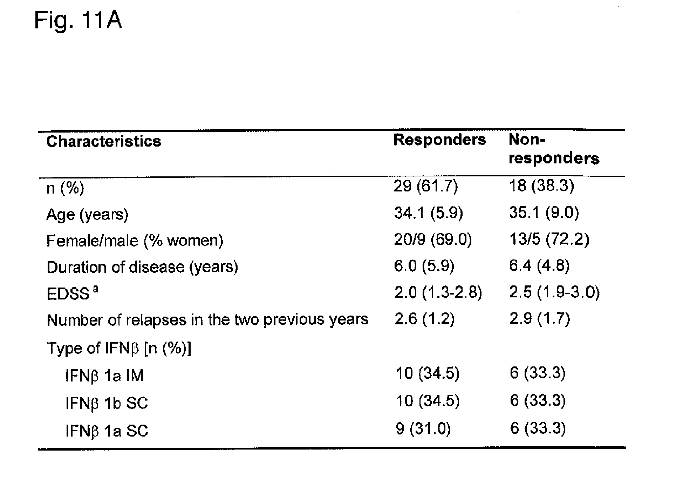

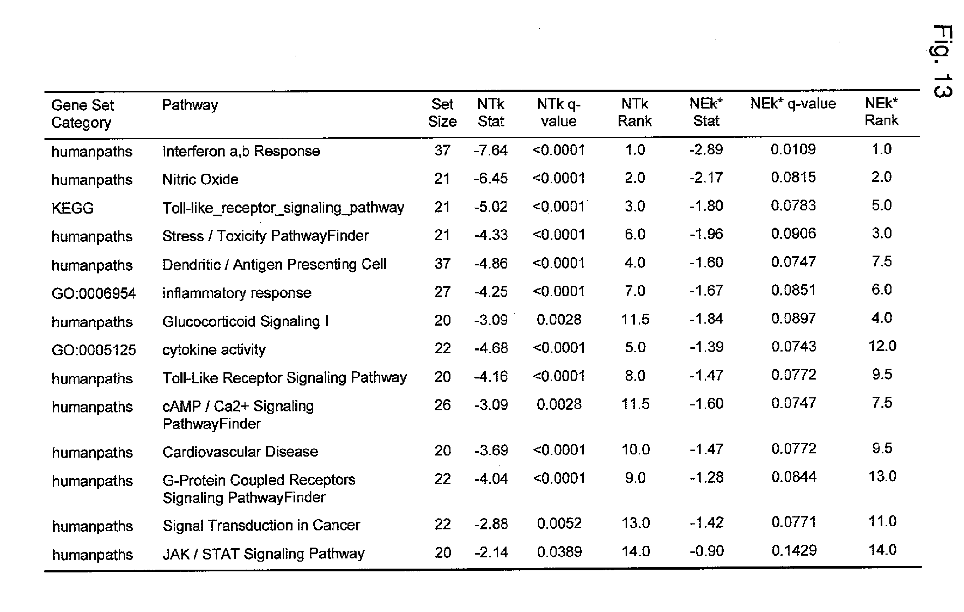

We first studied differentially expressed genes in PBMC from RRMS patients before initiating treatment with IFNβ, in order to identify the preexisting gene expression signatures that distinguish responders from non-responders. Demographic and baseline clinical characteristics of the 47 MS patients are summarized in **Figure 11A****.** To exclude a confounding role of neutralizing antibodies (NABs) against IFNβ, we measured NABs at baseline and following 12 and 24 months. None of the patients was positive for NABs at baseline. Two patients in the non-responder group (11.1%) and one patient in the responder group (3.4%) developed NABs at 12 or 24 months (Fisher's Exact Test: p=0.549). Since expression analyses focused on the baseline- and 3 months treatment time points and since the presence of NABs was not associated with the response to IFNβ, all patients were analyzed. We found 47 genes differentially expressed (p<0.05) at baseline between responders and non-responders (**Figure 12**). The vast majority of these genes were overexpressed in patients subsequently non-responsive to IFNβ. Functional annotation revealed the type I IFN signaling pathway as the most significantly associated with the non-responder "phenotype" (**Figure 13**). Up to 23% of genes differentially expressed at baseline are known to be predominantly or selectively induced by type I IFNs⁹⁻¹³. Differentially expressed genes were validated by real time RT-PCR, and as shown in **Figure 9****,** the majority of the differentially expressed genes were confirmed with values ranging from 1.6 to 5-fold overexpression in non-responders. These results point to preexisting differences between responders and non-responders in the activation of the type I IFN signaling pathway. An activated type I IFN signalling pathway is associated with lack of response to IFNβ. We next analyzed changes in gene expression during the first three months of IFNβ treatment by comparing 3 months and baseline expression levels in responders and non-responders. **Figure 14** lists the 43 genes that were found differentially expressed. Interestingly, the highest differences were obtained for genes predominantly or selectively induced by type I IFNs, and data were again confirmed by real time RT-PCR in a subgroup of genes. For the majority of them, a strong and significant induction was observed in treatment responders as reflected by fold changes from 3 to over 120 (**Figure 10**). Conversely, in non-responders the magnitude of changes in gene expression induced by IFNβ was much lower or absent and did not reach statistical significance for any gene. These findings indicate that non-responders have an activated type I IFN system in peripheral blood cells that is refractory to exogenous IFNβ.

### Type I IFN-induced genes are among the best treatment response predictors

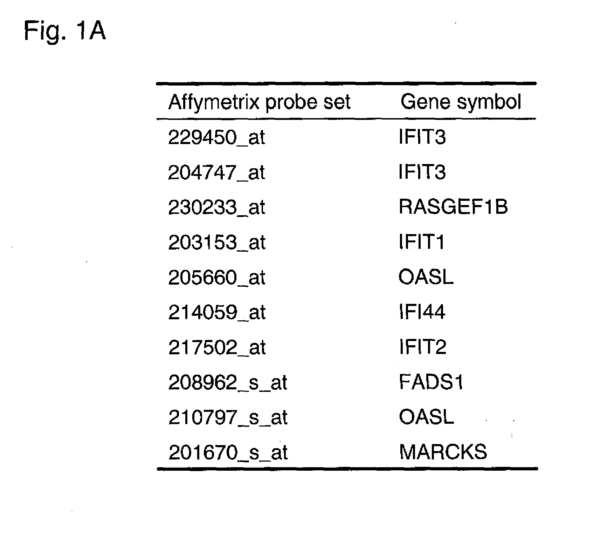

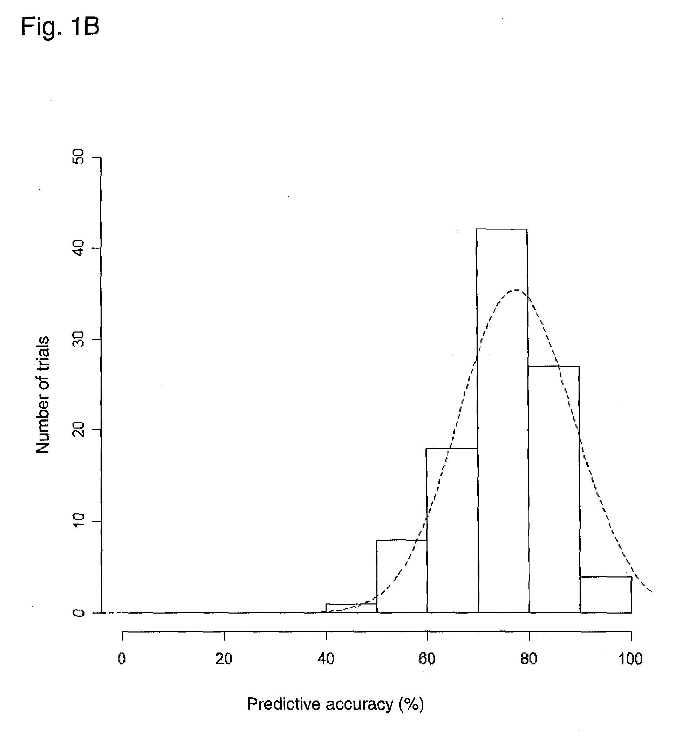

One objective of our study was the identification of differentially expressed genes that would allow to predict the response status to IFNβ after 24 months within the first 3 months of treatment. A prediction algorithm was applied comparing several predictors in a process that was cross-validated, as described under Methods. The "k-nearest neighbor with one neighbor" represented the predictor with the lowest misclassification error. **Fig. 1A** lists the 8 best discriminating genes between responders and non-responders obtained with this predictor. Interestingly, 5 of the 8 best predictor genes (*IFITI-3*, *IF144*, and *OASL*) are known to be selectively induced by type I IFNs. A mean predictive accuracy close to 80% was obtained with these 8 genes after randomly applying 100 times the k-nearest neighbor with one neighbor (**Fig. 1B**) indicating that the clinical response to IFNβ can indeed be predicted early.

### IFNβ non-responders show increased STAT1 phosphorylation and type I IFN receptor α expression in monocytes

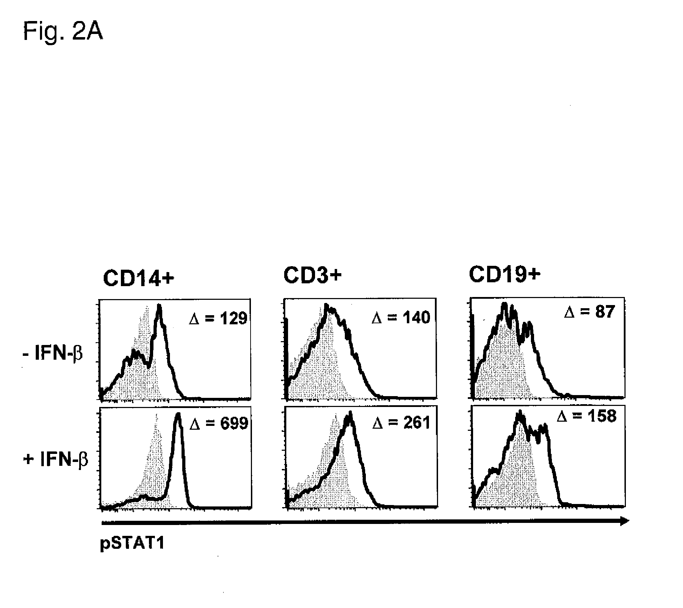

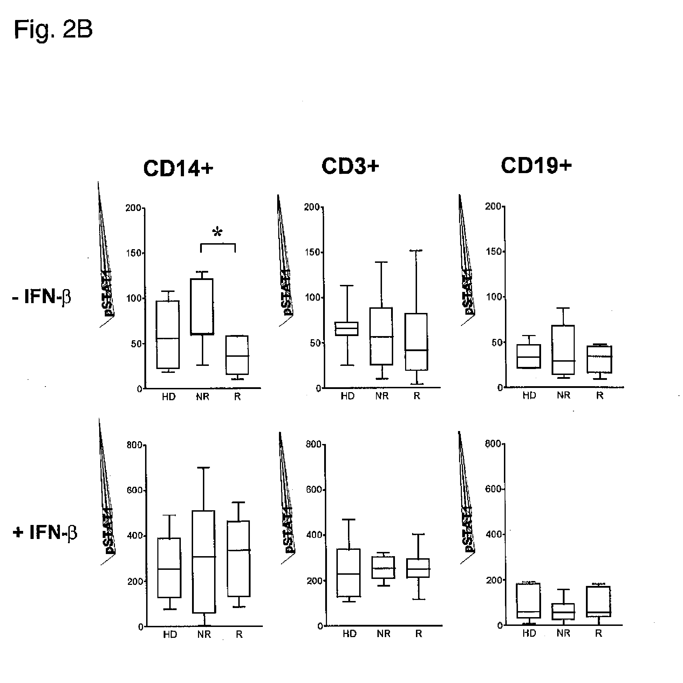

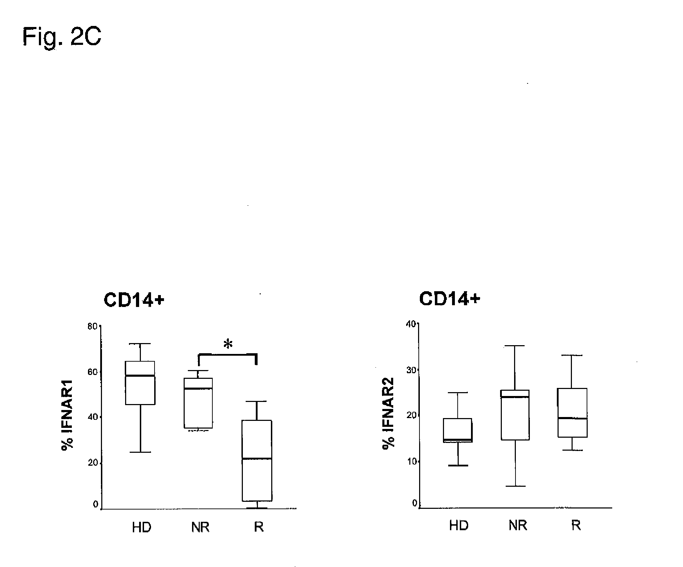

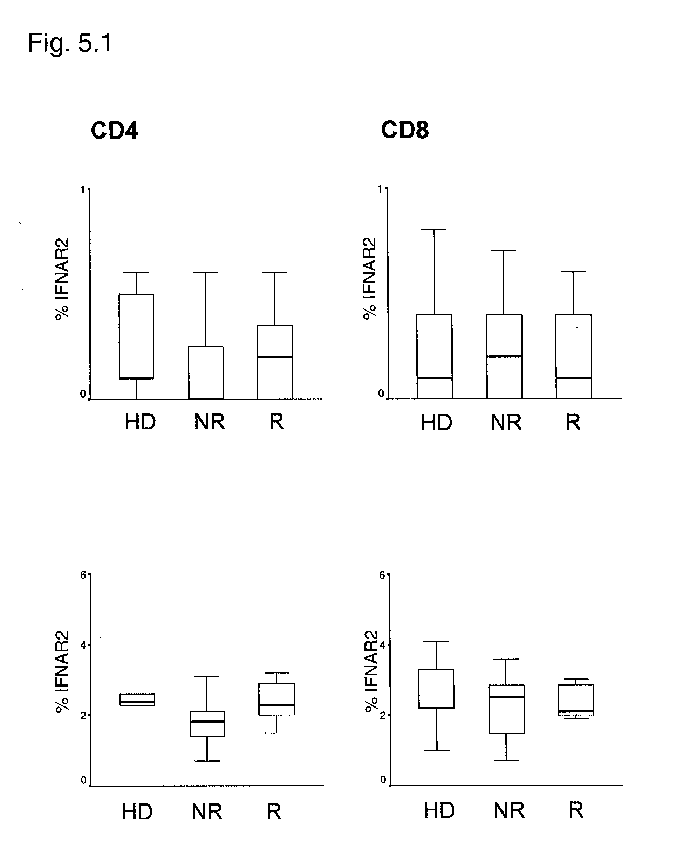

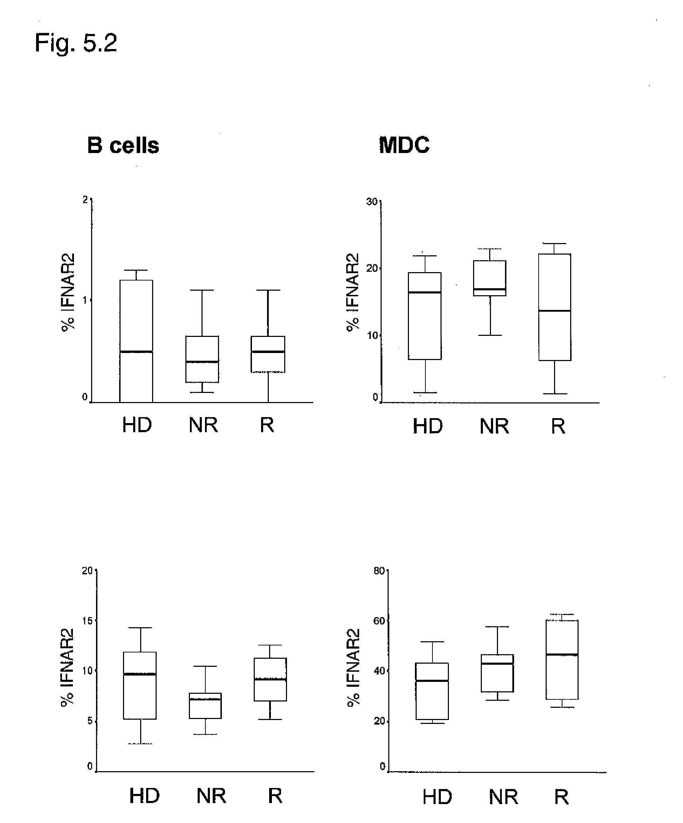

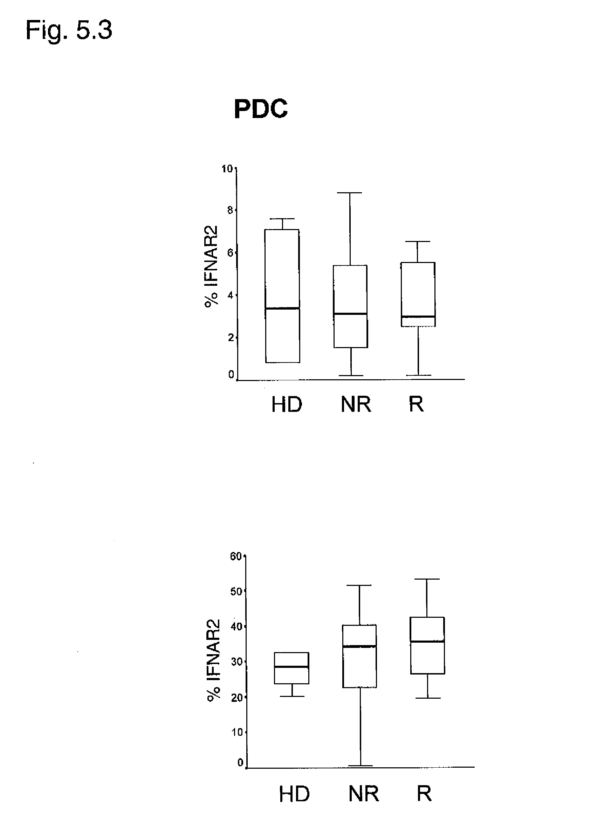

In order to understand the functional implications of our observations we performed additional experiments in PBMC obtained at baseline from responders and non-responders. Type I IFN-mediated activation of its receptor (IFNAR) activates Jak protein tyrosine kinases, which in turn phosphorylate STAT proteins including STAT1. Hence, we analysed phosphorylated STAT1 (p-STAT1) protein by flow cytometry as a marker for IFN-induced response at the single-cell level. As depicted in **Fig. 2A****,** IFNβ stimulation of PBMC led to a substantial increase of p-STAT1 levels in monocytes, T cells and B cells. Monocytes showed the highest IFNβ-induced p-STAT1 levels. There were no differences in IFNβ -induced p-STAT1 levels between groups indicating that the type I IFN receptor signaling machinery is not impaired in non-responders (**Fig. 2B**, lower panels). However, p-STAT1 baseline levels were significantly higher in monocytes derived from non-responders compared with responders (**Fig. 2B**, upper panels), while no differences were observed in T -and B cells. Of note, comparisons between baseline and IFNβ-induced p-STAT1 levels in each group revealed a stronger and statistically significant induction of p-STAT1 levels by IFNβ in responders [magnitude of the increase (Δ)=9; p=0.01, Wilcoxon Signed Ranks Test] and healthy donors (Δ=4.5; p=0.04) compared with non-responders (Δ=4.5; p=0.06).These results are in line with the fold change values observed for STAT1 gene expression levels when comparing treated and baseline samples between responders and non-responders (**Figure 10**) indicating an activated type I IFN system in monocytes prior to therapy with IFNβ and a lack of further inducibility in treatment non-responders. Consistent with this notion, we found a significantly elevated surface expression of IFNAR1, one component of the type I IFN surface receptor, to which all type I IFNs bind, on monocytes of non-responders (Fig. 2C). IFNAR2, the other molecule in the type I IFN receptor heterodimer, was not elevated on monocytes, and neither IFNAR1 nor IFNAR2 expression were altered on T-, B, or dendritic cells (DC) (**Fig. 5**).

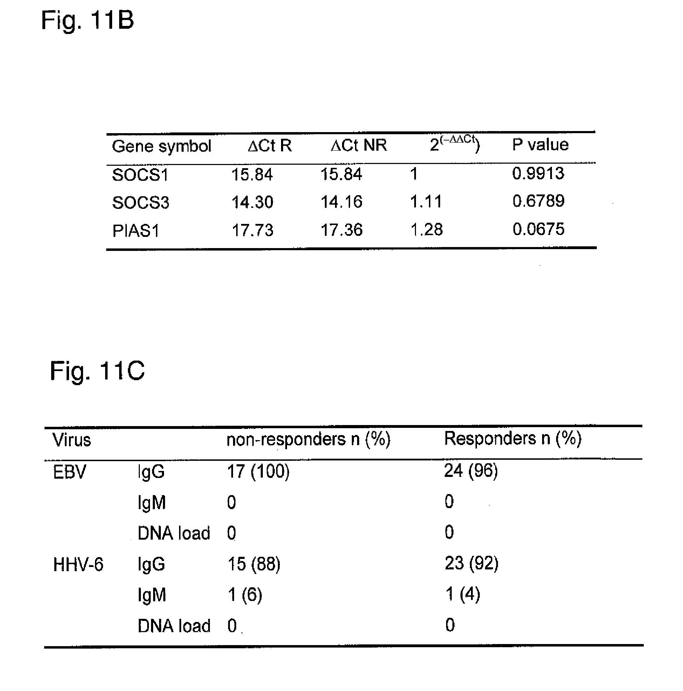

In further functional studies, we addressed whether the above alterations in the type I IFN pathway are due to lower expression of negative type I IFN regulators, suppressors of cytokine signalling 1 (*SOCS1*) and 3 *(SOCS3)* and protein inhibitor of activated STAT1 (*PIAS1*), which all negatively regulate the Jak/STAT signaling pathway. As shown in **Figure 11B****,** baseline expression levels of *SOCS1*, *SOCS3*, and *PIAS1* were similar between the two groups, ruling out a deficient expression of these genes as a cause of the activation of the type I IFN pathway in non-responders.

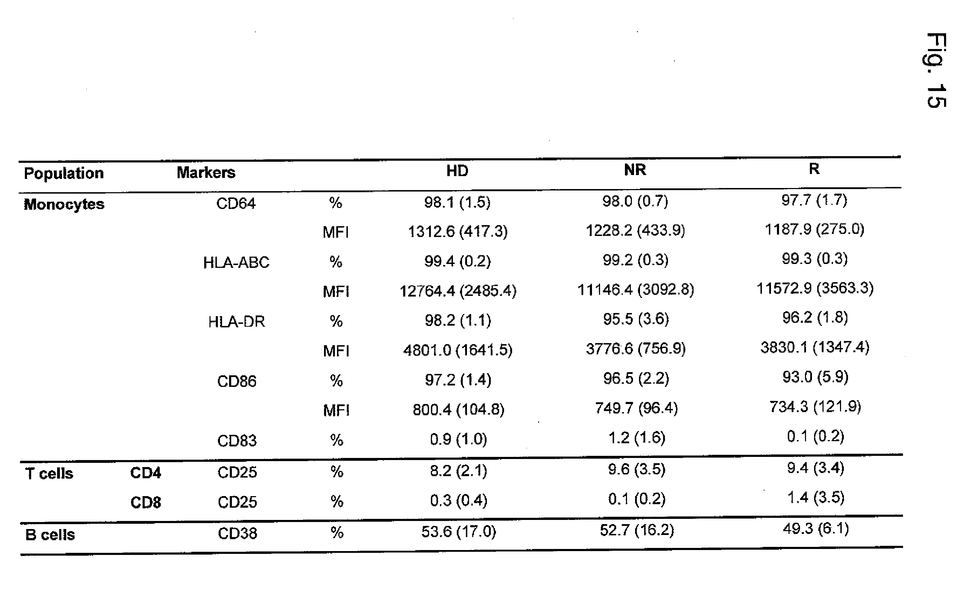

As a next step, we examined whether overexpression of type I IFN-responsive genes in non-responders occurs in the context of global activation of monocytes alone or together with other immune cells. As shown in **Figure 15****,** no significant differences in the expression of activation markers were observed between groups for monocytes or any other of the analyzed PBMC populations. CD83, which is expressed by 1.2% of monocytes in non-responders, and 0.1% of responders and was also transcribed at higher levels at baseline in non-responders (**Figure 12**) indicates, however, that a larger fraction of monocytes in non-responders have received a differentiating stimulus towards DC.

### Innate immune stimulation elicits higher type I IFN responses in IFNβ non-responders

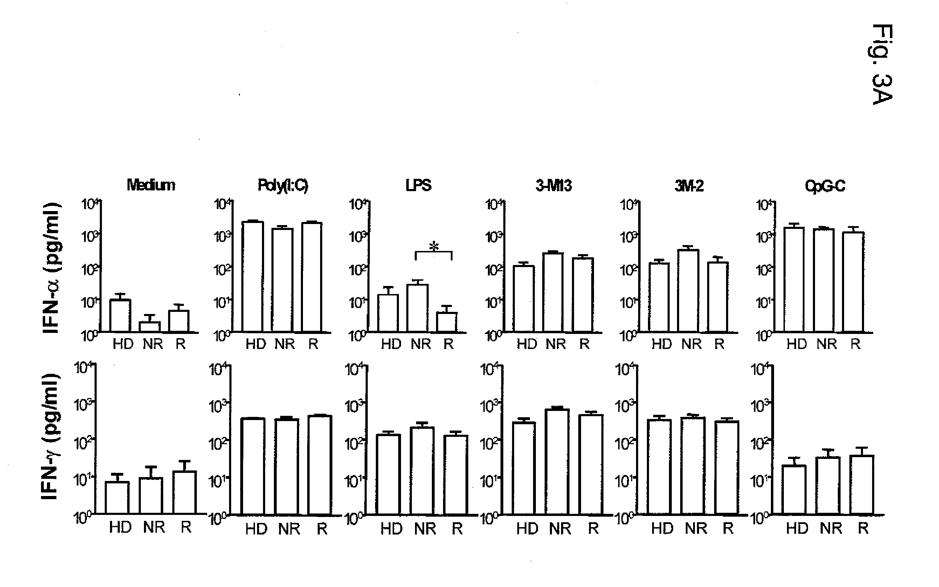

Many of the genes that are transcribed at higher levels at baseline in non-responders (*IL1B, TNF*, *STAT1*, *CXCL10, PTX3, CCR1*) suggest increased activation of the innate immune system. Microbial pathogens are strong stimuli for the innate immune system via a number of different receptors, among them toll-like receptors (TLRs). Following engagement of several TLRs type I IFNs act as major mediators of innate immune responses. TLR-induced type I IFN production are also believed to play an important role in triggering and sustaining autoimmune responses in systemic lupus erythematosus ^{14,15}, but also in MS¹⁶. To test whether the IFN signature in IFNβ non-responders was associated with an increased production of IFNs following stimulation with TLR agonists mimicking viral or bacterial products, we stimulated PBMC with classical TLR3- (polyI:C), TLR4- (LPS), TLR7- (3-M13), TLR8- (3M-2), and TLR9 (CpG-C) agonists and analyzed the supernatants for IFNα and IFNγ levels. As shown in **Fig. 3A****,** all TLR ligands elicited a robust IFN response. Interestingly, non-responders showed significantly higher levels of IFNα only following LPS stimulation compared with responders. This increased type I IFN production was most likely mediated by the monocyte lineage since we did not detect differences in the production of IFNγ following LPS stimulation. Also TLR4 expression by plasmacytoid DC, one of the major cell sources of IFNα, is low or absent¹⁷. These data suggest that non-responders differ from responders in their increased type I IFN response to TLR4 stimulation.

### Serum type I IFN bioactivity is increased in non-responders to IFNβ

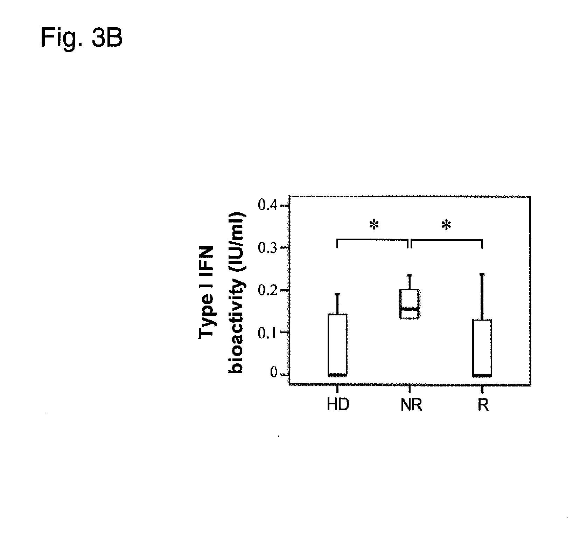

We next evaluated whether the baseline type I IFN signature observed in non-responders was associated with increased levels of type I IFNs. A gene reporter bioassay specific for type I IFNs was used to quantify type I IFN bioactivity in serum samples from patients and healthy donors. Although overall low, type I IFN bioactivity was significantly higher in non-responders to IFNβ compared with responders and healthy donors (**Fig. 3B**). These results suggest the presence of increased endogenous production of type I IFNs in non-responders as the cause of the baseline type I IFN signature observed in these patients. While we do not know the natural stimuli leading to increased type I IFN serum levels in non-responders, it is unlikely that Epstein Barr virus (EBV) or human herpes virus 6 (HHV-6), two viruses that are being discussed as etiological factors in MS, are responsible for these observations. Baseline EBV and HHV-6 DNA load was determined by quantitative real-time PCR. Neither responders nor non-responders showed detectable EBV or HHV-6 DNA levels in the PBMC or IgM seropositivity at baseline, and the IgG positivity for both viruses did not differ (**Figure 11C**)**.** These results make reactivation of a viral infection by EBV and HHV-6 unlikely as the cause of the differences of the type I IFN signaling pathway in both groups.

### Increased activation of myeloid dendritic cells in IFNβ non-responders

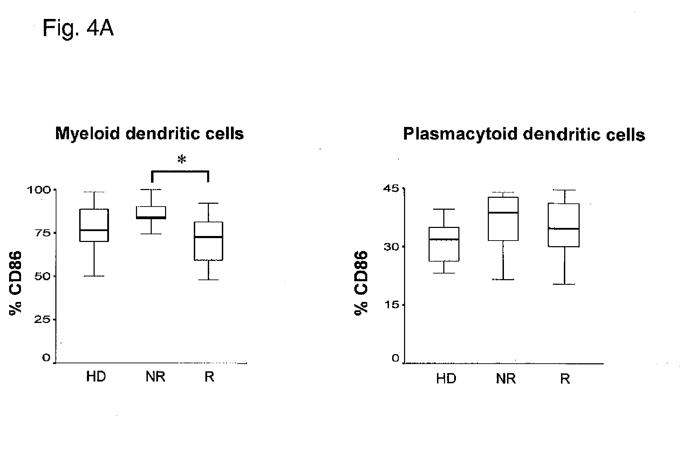

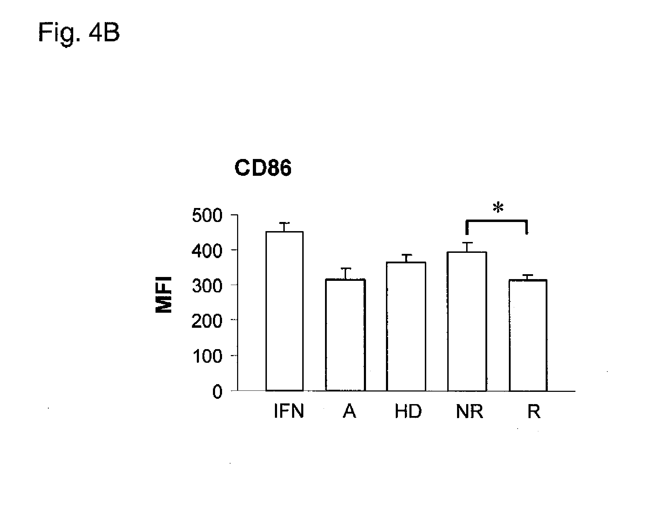

Type I IFNs induce activation and maturation of DC. To evaluate whether DC from non-responders differ in their activation status from responders, the expression of several activation- and maturation markers was determined on myeloid and plasmacytoid DC. As mentioned before, the higher fraction of monocytes expressing CD83 (**Figure 15**) already provided a hint of monocyte differentiation toward DC, and myeloid DC from non-responders showed a significant increase in CD86 expression compared to responders (**Fig. 4A****)**, whereas CD80 and CD83 were expressed at similar levels (**Figure 12**). These results suggest that myeloid DC from non-responders are more activated at baseline compared with responders.
It was next tested whether serum from non-responders favors differentiation of monocytes into DC by cultivating purified monocytes from healthy controls with baseline serum from responders, non-responders, and healthy donors. Consistent with the above findings serum from non-responders induced a significant increase in CD86 expression by monocytes after 3 days of culture when compared with responders, in whom levels of CD86 expression were similar to those found with autologous serum (**Fig. 4B**)**.** An increase of CD83 expression on monocytes was also observed upon incubation with serum from non-responders, but the difference did not reach statistical significance (**Figure 13**). Finally, no differences between groups were observed with respect to CD80 and HLA-DR expression. These results suggest the presence of soluble factors in serum from non-responders that induce monocyte differentiation.

### PBMC from patients, who will not respond to treatment, are characterized by a Th1 type immune response

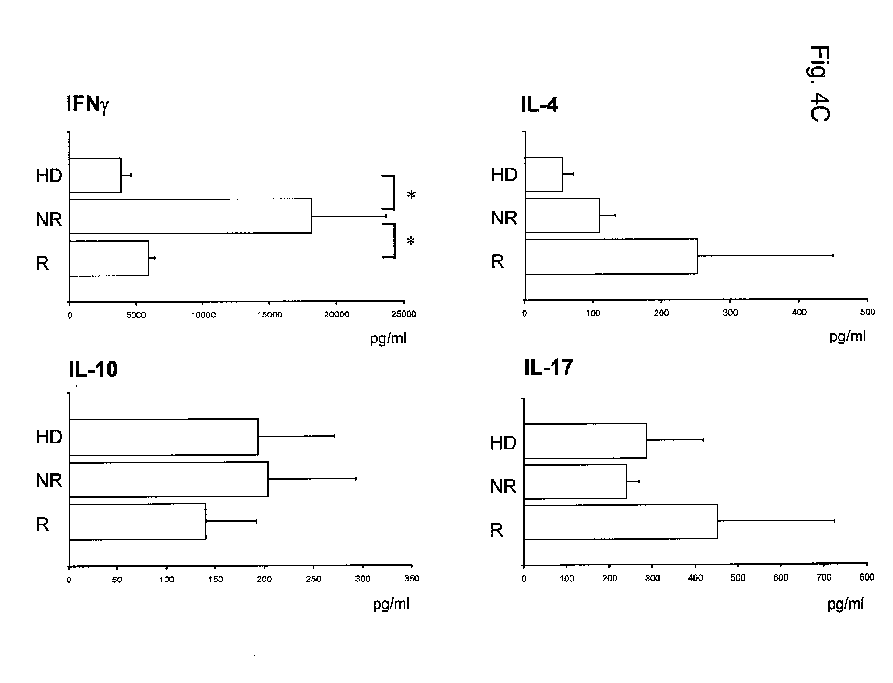

Type I IFNs play an important role in differentiation of T cells towards a T helper 1 (Th1) type proinflammatory immune response via STAT4 activation¹⁸⁻²⁰. To address whether T cell differentiation at baseline is skewed towards either proinflammatory (Th1, Th17) or immunomodulatory phenotypes in IFNβ non-responders, we measured the production of IL-4, IL-10, IL-17, and IFNγ in PBMC culture supernatants after mitogen stimulation. As shown in **Fig. 4C****,** a stronger Th1 type immune response was observed in PBMC from non-responders, as reflected by the significant increase in IFNγ production in non-responders versus responders and healthy donors. No significant differences between groups were observed for IL-4, IL-10, and IL-17.

### Explanation of the Figures:

**Figure 1****.** A set of predominantly type I IFN-induced genes are among the best predictors of response to IFNβ in the first 3 months of treatment.
   (**A**) List of genes discriminating best between responders and non-responders. A total of 6,410 genes from the initial list of 54,675 passed the non-specific filtering in their expression between baseline and three months. After applying a prediction algorithm to this set of genes, the k-nearest neighbor with one neighbor and with 8 genes (10 probe sets) were selected as the method and number of genes that had associated the lowest misclassification error (ε=0.3083). This misclassification error was computed as the mean of 10 cross-validation steps in which 9/10th of data were used as training data, i.e. to build a predictor, and the left out samples used as validation data, that is, to compute the misclassification error.
   (**B**) Histogram showing predictive accuracy of the best predictive genes after random partitions. To compute the histogram, an iterative method of 100 random divisions was applied to the original sample and filtered genes. Each random division selected a balanced set of 3/4th parts of the patients to build a predictor using the k-nearest neighbor method with one neighbor and 8 genes (10 probe sets). This predictor was applied to the remaining 1/4th part of patients to compute mean predictive accuracy over the 100 trials. The mean predictive accuracy of all random divisions was 78%, which we used to generate a normal approximation to predictive data, plotted in the figure as a dotted curve.
**Figure 2**. Selective increase of baseline STAT1 phosphorylation and IFNAR1 expression in CD14+ monocytes derived from IFNβ non-responders. (**A**) Baseline and IFNβ-induced levels of phosphorylated STAT1 (p-STAT1) protein were quantified by intracellular flow cytometry in CD14+ monocytes, CD3+ T cell and CD19+ B cells. IFNβ-1a stimulation led to a substantial increase of p-STAT1 levels in CD14+ monocytes, CD3+ T cell and CD19+ B cells. Monocytes showed the highest IFNβ induced p-STAT1 levels. (**B**) Phosphorylated STAT1 baseline levels are significantly higher in monocytes derived from non-responders compared with responders (*p=0.01, Mann Whitney U test). HD: healthy donors (n=10). NR: non-responders (n=10). R: responders (n=10). (**C**) Boxplots showing mean percentage of positive cells expressing IFNAR1 and IFNAR2 in non-responders (NR, n=11), responders (R, n=11), and healthy donors (HD, n=9). Baseline IFNAR1 expression by monocytes from non-responders is significantly higher compared with responders (*p=0.008, Mann Whitney U test) and similar to healthy donors.
**Figure 3****.** Type I IFN secretion of monocytes upon innate immune stimulation and serum type I IFN bioactivity are increased in IFNβ non-responders. (**A**) Type I (IFNα2) and type II (IFNγ) IFN production in PBMC following TLR stimulation. PBMC were stimulated with TLR3 (Poly(I:C); 25 mg/ml), TLR4 (LPS; 1 mg/ml), TLR7 (3M-13; 1 mg/ml), TLR8 (3M-2; 1 mg/ml), and TLR9 (CpG-C; 500mM) agonists in duplicates for 48 hours. Cytokine levels in supernatants were quantified by ELISA. Results are expressed as mean values (standard error of the mean, SEM). IFNα2 production following LPS stimulation is substantially higher in non-responders compared to responders (*p=0.03, Mann Whitney U test). There were no statistically significant differences in cytokine responses to other TLR agonists between healthy donors and MS patients and between IFNβresponders and non-responders. (**B**) Quantification of type I IFN bioactivity. A pro-monocytic cell line was transferred to a 96 well plate and then 25 µl of serum samples from MS patients and healthy donors were placed in individual wells and tested in duplicate for type I IFN bioactivity. Plates were finally read in a luminometer after incubation at 37°C for 17 hours and type I IFN bioactivity quantitated by extrapolation from a standard curve generated with IFNα-2b (range 1.5 to 25 IU/ml). Type I IFN bioactivity in non-responders is significantly higher compared with responders and healthy donors (*p=0.04 for both groups, Mann Whitney U test). HD: healthy donors (n=10). NR: non-responders (n=10). R: responders (n=10).
**Figure 4****.** In treatment non-responders myeloid DC are more activated, serum induces upregulation of CD86 in monocytes, and PBMC are characterized by a Th1 type of immune response. (**A**) Bloxplots showing baseline expression of CD86 in myeloid and plasmacytoid DC. PBMC from responders and non-responders to IFNβ were stained with monoclonal antibodies against CD11c, HLA-DR, CD86, and lineage cocktail. Myeloid DC were identified as lineage-HLADR+CD11c+ cells, whereas plasmacytoid DC were lineage-HLADR+CD11c-. Results are expressed as mean percentage of positive cells for each marker. CD86 expression is increased in myeloid DC from non-responders compared with responders (*p=0.006, Student-t-test). HD: healthy donors (n=9). NR: non-responders (n=10). R: responders (n=10). (**B**) Induction of DC differentiation from monocytes by serum from responders and non-responders. CD14+ purified monocytes were cultured for 3 days in the following conditions: IFNβ (100 IU/ml), autologous serum, serum from responders, serum from non-responders, or serum from healthy donors. Expression of CD86 was determined by flow cytometry. Results are expressed as mean values (SEM) of MFI. CD86 expression by monocyte derived DC is increased following incubation with serum from non-responders compared with responders (*p=0.02, Student-t-test). IFN: IFNα (n=2). A: autologous serum (n=5). HD: serum from healthy donors (n=6). NR: serum from non-responders (n=10). R: serum from responders (n=10). MFI: mean fluorescence intensity. (**C**) Quantification of cytokines in culture supernatants. PBMC obtained at baseline were stimulated with PMA (50 ng/ml) and ionomycin (1 µg/ml) for 24 hours. Levels of IL-4, IL-10, and IFNγ were measured in culture supernatants by ELISA (IL-17) and cytometric bead arrays (IL-4, IL-10, and IFNγ). Results are expressed as mean values (SEM). IFNγ production in culture supernatants from non-responders is significantly higher compared with responders and healthy donors (*p=0.008 for both groups, Mann Whitney U test). HD: healthy donors (n=5). NR: non-responders (n=5). R: responders (n=5).
**Figure 5****:** Expression of surface IFNAR1 and IFNAR2 in different cell populations. Boxplots showing mean percentage of positive cells expressing IFNAR1 and IFNAR2 in non-responders (NR, n=11), responders (R, n=11), and healthy donors (HD, n=9). No significant differences in the surface expression of IFNAR1 and IFNAR2 were observed between groups. MDC: myeloid dendritic cells. PDC: plasmacytoid dendritic cells.

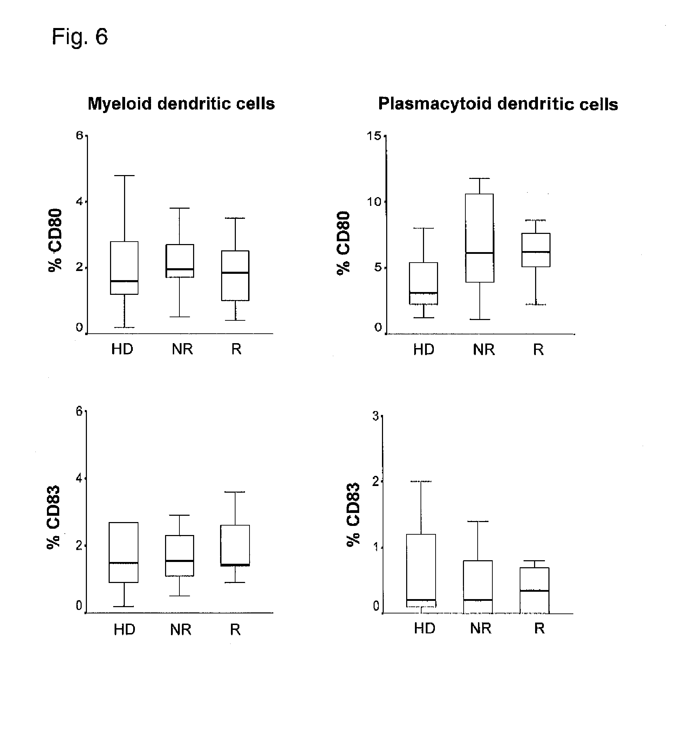

**Figure 6****.** Bloxplots showing expression of activation and maturation markers in myeloid and plasmacytoid DC. Baseline PBMC from responders and non-responders to IFNβ were stained with monoclonal antibodies against CD11c, HLA-DR, CD80, CD83, and lineage cocktail. Myeloid and plasmacytoid DC were identified as described under Methods. Results are expressed as mean percentage of positive cells for each marker. No differences in the expression of CD80 and CD83 were observed between groups for any of the DC subsets. HD: healthy donors (n=9). NR: non-responders (n=10). R: responders (n=10).

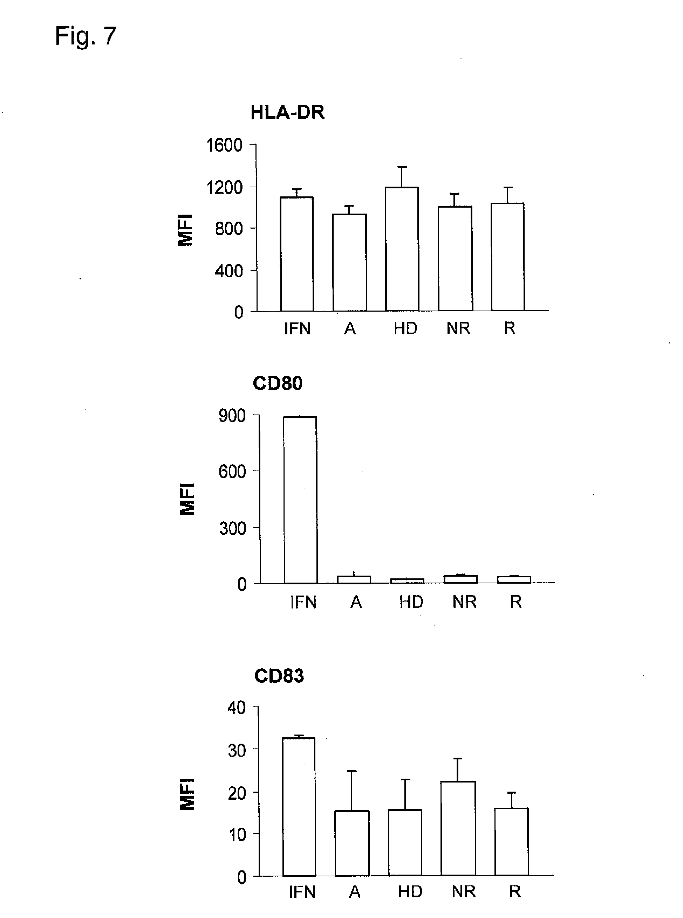

**Figure 7****.** Induction of DC differentiation from monocytes by serum. CD 14+ purified monocytes were cultured for 3 days as described under Methods. Expression of HLA-DR, CD80, and CD83 was performed by flow cytometry. Results are expressed as mean values (SEM) of MFI. No statistically significant differences were observed between groups for any of the markers. IFN: IFNα (n=2). A: autologous serum (n=5). HD: serum from healthy donors (n=6). NR: serum from non-responders (n=10). R: serum from responders (n=10). MFI: mean fluorescence intensity.
**Figure 8****.** Flow chart summarizing the different steps undertaken in the processing of microarray data. Patients were followed for at least 24 months after initiation of IFNβ treatment and then classified into responders and non-responders based on stringent clinical criteria. Data from patients were obtained at baseline (t=0) and after 3 months of treatment (t=3). Analysis was performed before treatment (t=0) comparing DEG between responders and non-responders (1), and after 3 months of treatment comparing differences in gene expression levels between 3 months and baseline samples (t=3-0) in responders and non-responders (2). Additionally, we applied a prediction algorithm to analyze whether changes in gene expression induced by IFNβ in the first 3 months of treatment could predict therapeutic outcome after 24 months (3). Lists of genes that discriminate between responders and non-responders were obtained at baseline (Supplementary Table 2) and after 3 months of treatment (Supplementary Table 3). Finally, a list of the best predictive genes of response to IFNβ was also obtained and depicted in Fig. 1. DEG obtained at baseline and changes in gene expression induced by IFNβ in the first 3 months of treatment were validated by real time RT-PCR (shown in Tables 1 and 2 respectively). IFNβ: interferon-beta. DEG: differentially expressed genes
**Figure 9** Validation by real time RT-PCR of baseline differentially expressed genes obtained with microarrays. Real time RT-PCR was performed in total RNA samples obtained at baseline from 24 responders and 16 non-responders to IFNβ. Among validated genes by RT-PCR, those genes either reported in the literature to be induced by type I IFNs or found significantly regulated by IFNβ in the present study are represented in bold, and those genes known to be induced predominantly or selectively by type I IFNs are represented by asterisk. ΔCt were computed as the mean value of all ΔCt expressions obtained for each gene in each group. 2^{(-ΔΔCt)}represents the fold change in gene expression normalized to the endogenous control and relative to responders. P value: refers to the p values obtained following comparisons of mean ΔCt between responders and non-responders by means of a Student-t-test. R: responders; NR: non-responders. Statistically significant p values are shown in bold.
**Figure 10****:** Validation by real time RT-PCR of changes in gene expression induced by IFNβ in the first three months of treatment. Gene expression levels were determined by real time RT-PCR at baseline (ΔCt t=0) and in the first 3 months of treatment (ΔCt t=3) in total RNA samples from 4 non-responders and 3 responders to IFNβ. ΔCt were computed as the mean value of all ΔCt expressions obtained for each gene in each group. 2^{(-ΔΔCt)} represents the fold change in gene expression normalized to the endogenous control and relative to responders. P value: refers to the p values obtained following comparisons of mean ΔCt between responders and non-responders by means of a paired t-test.
**Figure 11****:** (**A**) Demographic and baseline clinical characteristics of MS patients responders and non-responders to IFNβ treatment. Data are expressed as mean (SD) unless otherwise stated. aData are expressed as mean (interquartile range). EDSS: Expanded Disability Status Scale. IM: intramuscular. SC: subcutaneous. (**B**): Baseline expression levels of negative regulators of the type I IFN signalling pathway in IFNβ responders and non-responders. Baseline gene expression levels of SOCS1, SOCS3, and PIAS1 were determined by real time RT-PCR in total RNA samples from 14 responders and 15 non-responders to IFNβ. ΔCt were computed as the mean value of all ΔCt expressions obtained for each gene in each group. 2^{(-ΔΔCt)} represents the fold change in gene expression normalized to the endogenous control and relative to responders. P value: refers to the p values obtained following comparisons of mean ΔCt between responders and non-responders by means of a Student-t-test. R: responders; NR: non-responders. (C): Prevalence of IgG and IgM serologies and DNA load for EBV and HHV-6 in responders and non-responders to IFNβ. IgG and IgM serologies for EBV and HHV-6 were measured in 17 non-responders and 25 responders. In seropositive patients, detection of viral genome was performed as described in Methods.
**Figure 12****:** Baseline differentially expressed genes between responders and non-responders obtained with microarrays. Genes are listed by p value ranking. To avoid repetition, designations and chromosome locations of probe sets corresponding to the same gene are shown once. Genes either reported in the literature to be induced by type I IFNs or found significantly regulated by IFNβ in the present study are represented in bold. Genes known to be induced predominantly or selectively by type I IFNs are represented by asterisk. LogFC: mean log fold change. *p-value: uncorrected p values. None of the p values was <0.05 after correction for multiple testing.
**Figure 13****:** Relevant pathways associated with the response to IFNβ obtained with "sigPathway". Statistically significant pathways are found through a statistical hypothesis testing framework proposed by Tian et al. (2005)31 for determining whether a specified group of genes for a pathway has a coordinated association with a phenotype of interest. The two null hypotheses are the following: the genes in a gene set show the same pattern of associations with the phenotype compared with the rest of the genes (Q1). The gene set does not contain any genes whose expression levels are associated with the phenotype of interest (Q2). Then, two statistics are developed, one for each hypothesis, and to get statistical significance each statistic is compared against the distribution under the null hypothesis. Set Size is the number of genes in the original list contained in the corresponding pathway. NTk Stat is the value of the statistic corresponding to hypothesis Q1. NTk q-value is the adjusted p-value corresponding to hypothesis Q1. NTk Rank is the rank corresponding to hypothesis Q1. NEk* Stat is the value of the statistic corresponding to hypothesis Q2. NEk* q-value is the adjusted p-value corresponding to hypothesis Q2. NEk* Rank is the rank corresponding to hypothesis Q2. The global ranking is obtained by adding up rank corresponding to hypotheses Q1 and Q2.
**Figure 14****:** Differentially expressed genes obtained with microarrays after 3 months of treatment with IFNβ. Genes are listed by p value ranking. Changes in the gene expression levels induced by IFNβ in the first 3 months of treatment were compared between responders (n=21) and non-responders (n=14). Genes represented in bold were also found differentially expressed at baseline. LogFC: mean log fold change. *P value: uncorrected p values. None of the p values was <0.05 after correction for multiple testing.
**Figure 15****:** Expression of activation markers in different PBMC populations from responders and non-responders to IFNβ. Expression of activation markers in monocytes, T cells, and B cells was determined by flow cytometry. For markers expressed in practically all the cells from a specific population, results are expressed as both percentage of positive cells and MFI. Data are depicted as mean values (standard deviation). HD: healthy donors (n=9). NR: non-responders (n=11). R: responders (n=11). %: percentage of positive cells. MFI: mean fluorescence intensity.

### References

1. The Interferon β Multiple Sclerosis Study Group. Interferon beta-1b is effective in relapsing-remitting multiple sclerosis. I. Clinical results of a multicenter, randomized, double-blind, placebo-controlled trial. Neurology 43, 655-661 (1993).
2. Jacobs, L.D. et al. Intramuscular interferon beta-1a for disease progression in relapsing multiple sclerosis. The Multiple Sclerosis Collaborative Research Group (MSCRG). Ann. Neurol. 39, 285-294 (1996).
3. PRISMS (Prevention of Relapses and Disability by Interferon beta-1a Subcutaneously in Multiple Sclerosis) Study Group. Randomised double-blind placebo-controlled study of interferon beta-1a in relapsing/remitting multiple sclerosis. Lancet 352, 1498-1504 (1998).
4. Rio, J. et al. Assessment of different treatment failure criteria in a cohort of relapsing-remitting multiple sclerosis patients treated with interferon beta: implications for clinical trials. Ann. Neurol. 52, 400-406 (2002).
5. Wandinger, K.P. et al. TNF-related apoptosis inducing ligand (TRAIL) as a potential response marker for interferon-beta treatment in multiple sclerosis. Lancet 361, 2036-2043 (2003).
6. Baranzini, S.E., et al. Transcription-based prediction of response to IFNbeta using supervised computational methods. PLoS Biol. 3, e2 (2005).
7. Soilu-Hanninen, M., Laaksonen, M., Hanninen, A., Eralinna, J.P. & Panelius, M. Downregulation of VLA-4 on T cells as a marker of long term treatment response to interferon beta-1a in MS. J. Neuroimmunol. 167, 175-182 (2005).
8. Minagar, A. et al. Saliva soluble HLA as a potential marker of response to interferon-beta1a in multiple sclerosis: a preliminary study. J. Neuroinflammation 4, 16 (2007).
9. Der, S.D., Zhou, A., Williams, B.R.G. & Silverman, R.H. Identification of genes differentially regulated by interferon α, β, or γ using oligonucleotide arrays. Proc. Natl. Acad. Sci. USA 95, 15623-15628 (1998).
10. Leszczyniecka, M., Su, Z.Z., Kang, D.C., Sarkar, D. & Fisher, P.B. Expression regulation and genomic organization of human polynucleotide phosphorylase, hPNPase(old-35), a Type I interferon inducible early response gene. Gene 316, 143-156 (2003).
11. Barnes, B.J. et al. Global and distinct targets of IRF-5 and IRF-7 during innate response to viral infection. J. Biol. Chem. 279, 45194-45207 (2004).
12. Kirou, K.A. et al. Coordinate overexpression of interferon-alpha-induced genes in systemic lupus erythematosus. Arthritis Rheum. 50, 3958-3967 (2004).
13. Helbig, K.J., Lau, D.T., Semendric, L., Harley, H.A. & Beard, M.R. Hepatology 42, 702-710 (2005).
14. Blanco, P., Palucka, A.K., Gill, M., Pascual, V. & Banchereau, J. Induction of dendritic cell differentiation by IFN-alpha in systemic lupus erythematosus. Science 294, 1540-1543 (2001).
15. Pascual, V., Farkas, L. & Banchereau, J. Systemic lupus erythematosus: all roads lead to type I interferons. Curr. Opin. Immunol. 18, 676-682 (2006).
16. van Baarsen, L.G., et al. A subtype of multiple sclerosis defined by an activated immune defense program. Genes Immun. 7, 522-531 (2006).
17. Kadowaki, N. et al. Subsets of human dendritic cell precursors express different toll-like receptors and respond to different microbial antigens. J. Exp. Med. 194, 863-869 (2001).
18. Cho, S.S., et al. Activation of STAT4 by IL-12 and IFN-alpha: evidence for the involvement of ligand-induced tyrosine and serine phosphorylation. J. Immunol. 157, 4781-4789 (1996).
19. Rogge, L.L., et al. The role of Stat4 in species-specific regulation of Th cell development by type I IFNs. J. Immunol.161, 6567-6574 (1998).
20. Biron, C.A. Interferons alpha and beta as immune regulators--a new look. Immunity14, 661-664 (2001).
21. Rio, J., et al. Defining the response to interferon-beta in relapsing-remitting multiple sclerosis patients. Ann. Neurol. 59, 344-352 (2006).
29. Radvanyi, L.G., Banerjee, A., Weir, M. & Messner, H. Low levels of interferon-alpha induce CD86 (B7.2) expression and accelerates dendritic cell maturation from human peripheral blood mononuclear cells. Scand. J. Immunol. 50, 499-509 (1999).
30. Le Bon, A., et al. Type I interferons potently enhance humoral immunity and can promote isotype switching by stimulating dendritic cells in vivo. Immunity 14, 461-470 (2001).
31. Brinkmann, V., Geiger, T., Alkan, S. & Heusser, C.H. Interferon alpha increases the frequency of interferon gamma-producing human CD4+ T cells. J. Exp. Med. 178, 1655-1663 (1993).
33. Irizarry, R.A., et al. Exploration, normalization, and summaries of high density oligonucleotide array probe level data. Biostatistics 4, 249-264 (2003).
34. Smyth, G.K. Linear models and empirical bayes methods for assessing differential expression in microarray experiments. Stat. Appl. Genet. Mol. Biol. 3, Article3 (2004).
35. Benjamini, Y. & Hochberg, Y. Controlling the false discovery rate: a practical and powerful approach to multiple testing. J. Roy. Stat. Soc. B. 57, 289-300 (1995).
36. Barrier, A., et al. Colon cancer prognosis prediction by gene expression profiling. Oncogene 24, 6155-6164 (2005).
37. Dudoit, S., Fridlyand, J. & Speed, T.P. Comparison of Discrimination Methods for the Classification of Tumors Using Gene Expression Data. J. Am. Stat. Assoc. 97, 77-87 (2002).
38. Tian, L., et al. Discovering statistically significant pathways in expression profiling studies. Proc. Natl. Acad. Sci. USA 102, 13544-13549 (2005).
39. Livak, K.J. & Schmittgen, T.D. Analysis of relative gene expression data using real-time quantitative PCR and the 2(-Delta Delta C(T)) Method. Methods 25, 402-408 (2001).
40. Lesinski, G.B., et al. Multiparametric flow cytometric analysis of inter-patient variation in STAT1 phosphorylation following interferon Alfa immunotherapy. J. Natl. Cancer Inst. 96, 1331-1342 (2004).
41. Dhodapkar, K.M., et al. Selective blockade of the inhibitory Fc{gamma} receptor (Fe{gamma}RIIB} in human dendritic cells and monocytes induces a type I interferon response program. J. Exp. Med. 204, 1359-1369 (2007).
42. Watzinger, F., et al. Real-time quantitative PCR assays for detection and monitoring of pathogenic human viruses in immunosuppressed pediatric patients. J. Clin. Microbiol. 42, 5189-5198 (2004).
43. Gneiss, C., et al. Differing immunogenic potentials of interferon beta preparations in multiple sclerosis patients. Mult. Scler. 12, 731-737 (2006).

## Claims

1. A method for predicting the treatment response of a multiple sclerosis patient to a type I IFN, comprising the steps of
(a) determining the expression level or activity of IFN type I or of at least one marker regulated by type I IFN in a sample obtained from said patient,
(b) comparing the level or activity to a standard,
(c) predicting the treatment response, wherein a treatment with a type I IFN is predicted to be ineffective in case the patient shows an altered level or activity.

2. A method for predicting the treatment response of a multiple sclerosis patient to a type I IFN, comprising the steps of
(a) determining the expression level or activity of at least one marker in a sample obtained from said patient,
(b) comparing the level or activity to a standard,
(c) predicting the treatment response, wherein a treatment with a type I IFN is predicted to be ineffective in case the patient shows an altered expression level or activity of the marker,
wherein the patient was not subjected to a type I IFN treatment prior to obtaining said sample, and/or wherein the method does not comprise a step of contacting said sample obtained from said patient with a type I IFN.

3. The method of any of the preceding claims, wherein the sample comprises PBMC and/or monocytes or is obtained from PBMC and/or monocytes.

4. The method of any of the preceding claims, wherein the type I IFN is β-INF.

5. The method of any of the preceding claims, wherein the marker is an mRNA, a cDNA, a gene or a protein.

6. The method of any of the preceding claims, wherein in step (a) the expression level of at least one mRNA is determined and in step (c) a treatment with a type I IFN is predicted to be ineffective in case the patient shows an increased expression level of the at least one mRNA.

7. The method of any of the preceding claims, wherein in step (a) the expression level of at least one marker is determined, the marker being selected from the group consisting of IFIT3, IFIT1, IFIT2, RASGEF1B, IFI44, FADS1, OASL and MARCKS, wherein the treatment is predicted to be ineffective in case the level is increased.

8. The method of any of the preceding claims, wherein in step (a) the expression level of at least one marker is determined, selected from the group consisting of RASGEF1B, IRF5, IFIT3, IL1RN, IFIT1, ATXN1, MARCKS, CXCL10, OASL, H1F0, STAT1, IFIT2, TNF, APOL6, IL1B, LOC129607, CCR1, OAS2, RSAD2, SASH1, DDX58, IL8, IER3, PBEF1, PTX3, HERC5, PNPT1, CD83, LOC389833, LDLR, ATF3, OAS3, PTGDS, IFI44, PARP9, ISG15, LILRA5, EGR3, SAT, LOC653071, CXCL2, RAD50, PPP1R15A, NFKBIZ, LOC653071, TMEM176B, CDC42, NFKBIZ, PPP1R15A and GPR109B.

9. The method of claim 1, wherein in step (a) at least one of the following is determined:
(a1) type I IFN secretion of monocytes upon innate immune stimuli via TLR4, wherein the treatment with a type I IFN is predicted to be ineffective in case the secretion is increased,
(a2) serum type I IFN bioactivity, wherein the treatment with a type I IFN is predicted to be ineffective in case the secretion is increased,
(a3) an activation status of myeloid DCs, wherein the treatment with a type I IFN is predicted to be ineffective in case the status is elevated,
(a4) the T helper cell response towards Th1, wherein the treatment with a type I IFN is predicted to be ineffective in case the cell response is preferentially skewed,
(a5) the level of phosphorylated STAT1, wherein the treatment with a type I IFN is predicted to be ineffective in case the level is increased.

10. The method of any of the preceding claims, wherein the treatment is predicted to be ineffective if the level or activity of the marker is increased by at least 10%.

11. A method for treating multiple sclerosis in a patient showing an altered type I IFN response profile, comprising the step of administering a substance for neutralizing type I IFNs.

12. The method of claim 10, wherein the substance is an antibody or an interferon inhibitor.

13. A micorarray for predicting the treatment response of a multiple sclerosis patient to a type I IFN, wherein the microarray comprises not more than 200 markers and comprises at least 3 markers for determining an altered type I IFN response profile.

14. A micorarray for predicting the treatment response of a multiple sclerosis patient to a type I IFN, comprising at least 3 markers selected from the group consisting of RASGEF1B, IRF5, IFIT3, IL1RN, IFIT1, ATXN1, MARCKS, CXCL10, OASL, H1F0, STAT1, IFIT2, TNF, APOL6, IL1B, LOC129607, CCR1, OAS2, RSAD2, SASH1, DDX58, IL8, IER3, PBEF1, PTX3, HERC5, PNPT1, CD83, LOC389833, LDLR, ATF3, OAS3, PTGDS, IFI44, PARP9, ISG15, LILRA5, EGR3, SAT, LOC653071, CXCL2, RAD50, PPP1R15A, NFKBIZ, LOC653071, TMEM176B, CDC42, NFKBIZ, PPP1R15A and GPR109B.

15. The use of a microarray of claim 11 or 12 for predicting the treatment response of a multiple sclerosis patient to a type I IFN.

16. A diagnostic kit comprising a microarray of claim 10 or 11.
